# EUROPÄISCHE PATENTSCHRIFT

(11) **EP 1 200 426 B1**
(45) Veröffentlichungstag und Bekanntmachung des Hinweises auf die Patenterteilung: **25.02.2004**
(21) Anmeldenummer: 00947925.4
(22) Anmeldetag: 04.07.2000
(51) Int. Cl.: C07D 409/12, A01N 43/653

(54) **SUBSTITUIERTE THIEN-3-YL-SULFONYLAMINO(THIO)CARBONYL-TRIAZOLIN(THI)ONE**
SUBSTITUTED THIENE-3-YL-SULFONYL AMINO(THIO)CARBONYL-TRIAZOLIN(THI)ONES
THIEN-3-YL-SULFONYLAMINO(THIO)CARBONYL-TRIAZOLIN(THI)ONES SUBSTITUEES

(30) Priorität: 15.07.1999 DE 19933260
(43) Veröffentlichungstag der Anmeldung: 02.05.2002
(73) Patentinhaber: Bayer CropScience AG, 40789 Monheim (DE)
(72) Erfinder: GESING, Ernst, Rudolf, F., D-40699 Erkrath-Hochdahl (DE); KLUTH, Joachim, D-40764 Langenfeld (DE); MÜLLER, Klaus-Helmut, D-40593 Düsseldorf (DE); DREWES, Mark, Wilhelm, D-40764 Langenfeld (DE); DAHMEN, Peter, D-41470 Neuss (DE); FEUCHT, Dieter, D-40789 Monheim (DE); PONTZEN, Rolf, D-42799 Leichlingen (DE)
(86) Internationale Anmeldenummer: PCT/EP2000/006276
(87) Internationale Veröffentlichungsnummer: WO 2001/005788

(56) Entgegenhaltungen:
- EP-A- 0 341 489
- EP-A- 0 482 349
- EP-A- 0 534 266
- WO-A-97/03980
- WO-A-97/16449
- WO-A-98/07721
- WO-A-98/24787

## Beschreibung

Die Erfindung betrifft neue substituierte Thien-3-yl-sulfonylamino(thio)carbonyltriazolin(thi)one, Verfahren und neue Zwischenprodukte zu ihrer Herstellung sowie ihre Verwendung als Herbizide.

Es ist bereits bekannt, daß bestimmte substituierte Thienylsulfonylamino(thio)-carbonyl-triazolin(thi)one herbizide Eigenschaften aufweisen (vgl. WO-A-97/16449, WO-A-98/24787). Die Wirkung dieser Verbindungen ist jedoch nicht in allen Belangen zufriedenstellend.

Es wurden nun die neuen substituierten Thien-3-yl-sulfonylamino(thio)carbonyltriazolin(thi)one der allgemeinen Formel (I) 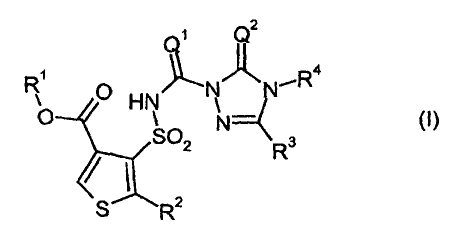 in welcher
- Q¹: für O (Sauerstoff) oder S (Schwefel) steht,
- Q²: für O (Sauerstoff) oder S (Schwefel) steht,
- R¹: für gegebenenfalls durch Cyano, Halogen oder C₁-C₄-Alkoxy substituiertes Alkyl mit 1 bis 6 Kohlenstoffatomen, für jeweils gegebenenfalls durch Cyano oder Halogen substituiertes Alkenyl oder Alkinyl mit jeweils 2 bis 6 Kohlenstoffatomen, für jeweils gegebenenfalls durch Cyano, Halogen oder C₁-C₄-Alkyl substituiertes Cycloalkyl oder Cycloalkylalkyl mit jeweils 3 bis 6 Kohlenstoffatomen in der Cycloalkylgruppe und gegebenenfalls 1 bis 4 Kohlenstoffatomen im Alkylteil, für jeweils gegebenenfalls durch Nitro, Cyano, Halogen, C₁-C₄-Alkyl oder C₁-C₄-Alkoxy substituiertes Aryl oder Arylalkyl mit jeweils 6 oder 10 Kohlenstoffatomen in der Arylgruppe und gegebenenfalls 1 bis 4 Kohlenstoffatomen im Alkylteil, oder für jeweils gegebenenfalls durch Nitro, Cyano, Halogen, C₁-C₄-Alkyl oder C₁-C₄-Alkoxy substituiertes Heterocyclyl oder Heterocyclylalkyl mit jeweils bis zu 6 Kohlenstoffatomen und zusätzlich 1 bis 4 Stickstoffatomen und/oder 1 bis 2 Sauerstoff- oder Schwefelatomen in der Heterocyclylgruppe und gegebenenfalls 1 bis 4 Kohlenstoffatomen im Alkylteil steht,
- R²: für Wasserstoff, Cyano, Nitro, Halogen, für jeweils gegebenenfalls durch Cyano, Halogen oder C₁-C₄-Alkoxy substituiertes Alkyl, Alkoxy, Alkylthio, Alkylsulfinyl oder Alkylsulfonyl mit jeweils 1 bis 6 Kohlenstoffatomen in der Alkylgruppe, oder für jeweils gegebenenfalls durch Cyano oder Halogen substituiertes Alkenyl, Alkinyl, Alkenyloxy oder Alkinyloxy mit jeweils 2 bis 6 Kohlenstoffatomen in der Alkenyl- oder Alkinylgruppe steht,
- R³: für Wasserstoff, Hydroxy, Mercapto, Amino, Cyano, Fluor, Chlor, Brom, Iod, für gegebenenfalls durch Fluor, Chlor, Brom, Cyano, C₁-C₄-Alkoxy, C₁-C₄-Alkyl-carbonyl oder C₁-C₄-Alkoxy-carbonyl substituiertes Alkyl mit 1 bis 6 Kohlenstoffatomen, für jeweils gegebenenfalls durch Fluor, Chlor und/oder Brom substituiertes Alkenyl oder Alkinyl mit jeweils 2 bis 6 Kohlenstoffatomen, für jeweils gegebenenfalls durch Fluor, Chlor, Cyano, C₁-C₄-Alkoxy oder C₁-C₄-Alkoxy-carbonyl substituiertes Alkoxy, Alkylthio, Alkylamino oder Alkyl-carbonylamino mit jeweils 1 bis 6 Kohlenstoffatomen in der Alkylgruppe, für Alkenyloxy, Alkinyloxy, Alkenylthio, Alkinylthio, Alkenylamino oder Alkinylamino mit jeweils 3 bis 6 Kohlenstoffatomen in der Alkenyl- oder Alkinylgruppe, für Dialkylamino mit jeweils 1 bis 4 Kohlenstoffatomen in den Alkylgruppen, für jeweils gegebenenfalls durch Methyl und/oder Ethyl substituiertes Aziridino, Pyrrolidino, Piperidino oder Morpholino, für jeweils gegebenenfalls durch Fluor, Chlor, Brom, Cyano und/oder C₁-C₄-Alkyl substituiertes Cycloalkyl, Cycloalkenyl, Cycloalkyloxy, Cycloalkylthio, Cycloalkylamino, Cycloalkylalkyl, Cycloalkylalkoxy, Cycloalkylalkylthio oder Cycloalkylalkylamino mit jeweils 3 bis 6 Kohlenstoffatomen in der Cycloalkyl- bzw. Cycloalkenylgruppe und gegebenenfalls 1 bis 4 Kohlenstoffatomen im Alkylteil, oder für jeweils gegebenenfalls durch Fluor, Chlor, Brom, Cyano, Nitro, C₁-C₄-Alkyl, Trifluormethyl, C₁-C₄-Alkoxy und/oder C₁-C₄-Alkoxy-carbonyl substituiertes Aryl, Arylalkyl, Aryloxy, Arylalkoxy, Arylthio, Arylalkylthio, Arylamino oder Arylalkylamino mit jeweils 6 oder 10 Kohlenstoffatomen in der Arylgruppe und gegebenenfalls 1 bis 4 Kohlenstoffatomen im Alkylteil steht,
- R⁴: für Wasserstoff, Hydroxy, Amino, Cyano, für C₂-C₁₀-Alkylidenamino, für gegebenenfalls durch Fluor, Chlor, Brom, Cyano, C₁-C₄-Alkoxy, C₁-C₄-Alkyl-carbonyl oder C₁-C₄-Alkoxy-carbonyl substituiertes Alkyl mit 1 bis 6 Kohlenstoffatomen, für jeweils gegebenenfalls durch Fluor, Chlor und/oder Brom substituiertes Alkenyl oder Alkinyl mit jeweils 2 bis 6 Kohlenstoffatomen, für jeweils gegebenenfalls durch Fluor, Chlor, Brom, Cyano, C₁-C₄-Alkoxy oder C₁-C₄-Alkoxy-carbonyl substituiertes Alkoxy, Alkylamino oder Alkyl-carbonylamino mit jeweils 1 bis 6 Kohlenstoffatomen in der Alkylgruppe, für Alkenyloxy mit 3 bis 6 Kohlenstoffatomen, für Dialkylamino mit jeweils 1 bis 4 Kohlenstoffatomen in den Alkylgruppen, für jeweils gegebenenfalls durch Fluor, Chlor, Brom, Cyano und/oder C₁-C₄-Alkyl substituiertes Cycloalkyl, Cycloalkylamino oder Cycloalkylalkyl mit jeweils 3 bis 6 Kohlenstoffatomen in der Alkylgruppe und gegebenenfalls 1 bis 4 Kohlenstoffatomen im Alkylteil, oder für jeweils gegebenenfalls durch Fluor, Chlor, Brom, Cyano, Nitro, C₁-C₄-Alkyl, Trifluormethyl und/oder C₁-C₄-Alkoxy substituiertes Aryl oder Arylalkyl mit jeweils 6 oder 10 Kohlenstoffatomen in der Arylgruppe und gegebenenfalls 1 bis 4 Kohlenstoffatomen im Alkylteil steht, oder
- R³ und R⁴: zusammen für gegebenenfalls verzweigtes Alkandiyl mit 3 bis 6 Kohlenstoffatomen stehen,
- sowie Salze dieser Verbindungen der Formel (I) -
gefunden.

Gesättigte oder ungesättigte Kohlenwasserstoffgruppierungen, wie Alkyl, Alkandiyl, Alkenyl oder Alkinyl, sind - auch in Verknüpfungen mit Heteroatomen, wie in Alkoxy - soweit möglich jeweils geradkettig oder verzweigt.

Gegebenenfalls substituierte Reste können einfach oder mehrfach substituiert sein, wobei bei Mehrfachsubstitution die Substituenten gleich oder verschieden sein können.

Bevorzugte Substituenten bzw. Bereiche der in den oben und nachstehend aufgeführten Formeln vorhandenen Reste werden im folgenden definiert.
- Q¹: steht bevorzugt für O (Sauerstoff) oder S (Schwefel).
- Q²: steht bevorzugt für O (Sauerstoff) oder S (Schwefel).
- R¹: steht bevorzugt für jeweils gegebenenfalls durch Cyano, Fluor, Chlor, Methoxy oder Ethoxy substituiertes Methyl, Ethyl, n- oder i-Propyl, n-, i-, s- oder t-Butyl, für jeweils gegebenenfalls durch Cyano, Fluor oder Chlor substituiertes Propenyl, Butenyl, Propinyl oder Butinyl, für jeweils gegebenenfalls durch Cyano, Fluor, Chlor, Methyl oder Ethyl substituiertes Cyclopropyl, Cyclobutyl, Cyclopentyl, Cyclohexyl, Cyclopropylmethyl, Cyclobutylmethyl, Cyclopentylmethyl oder Cyclohexylmethyl, für jeweils gegebenenfalls durch Cyano, Fluor, Chlor, Brom, Methyl, Ethyl, n- oder i-Propyl, Trifluormethyl, Methoxy, Ethoxy, n- oder i-Propoxy, Difluormethoxy oder Trifluormethoxy substituiertes Phenyl, Phenylmethyl oder Phenylethyl, oder für jeweils gegebenenfalls durch Cyano, Fluor, Chlor, Brom, Methyl, Ethyl, n- oder i-Propyl, Methoxy, Ethoxy, n- oder i-Propoxy substituiertes Heterocyclyl oder Heterocyclylmethyl, wobei die Heterocyclylgruppe jeweils aus der Reihe Oxetanyl, Thietanyl, Furyl, Tetrahydrofuryl, Thienyl, Tetrahydrothienyl ausgewählt ist.
- R²: steht bevorzugt für Wasserstoff, Cyano, Fluor, Chlor, Brom, für jeweils gegebenenfalls durch Cyano, Fluor, Chlor, Methoxy oder Ethoxy substituiertes Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, oder für jeweils gegebenenfalls durch Cyano, Fluor oder Chlor substituiertes Propenyl, Butenyl, Propinyl, Butinyl, Propenyloxy, Butenyloxy, Propinyloxy oder Butinyloxy.
- R³: steht bevorzugt für Wasserstoff, Hydroxy, Mercapto, Amino, Cyano, Fluor, Chlor, Brom, für jeweils gegebenenfalls durch Fluor, Chlor, Cyano, Methoxy, Ethoxy, n- oder i-Propoxy, Acetyl, Propionyl, n- oder i-Buyroyl, Methoxycarbonyl, Ethoxycarbonyl, n- oder i-Propoxy-carbonyl substituiertes Methyl, Ethyl, n- oder i-Propyl, n-, i-, s- oder t-Butyl, für jeweils gegebenenfalls durch Fluor, Chlor und/oder Brom substituiertes Ethenyl, Propenyl, Butenyl, Ethinyl, Propinyl oder Butinyl, für jeweils gegebenenfalls durch Fluor, Chlor, Cyano, Methoxy, Ethoxy, n- oder i-Propoxy, Methoxycarbonyl, Ethoxycarbonyl, n- oder i-Propoxy-carbonyl substituiertes Methoxy, Ethoxy, n- oder i-Propoxy, n-, i-, s- oder t-Butoxy, Methylthio, Ethylthio, n- oder i-Propylthio, n-, i-, s- oder t-Butylthio, Methylamino, Ethylamino, n- oder i-Propylamino, n-, i-, s- oder t-Butylamino, Acetylamino oder Propionylamino, für Propenyloxy, Butenyloxy, Ethinyloxy, Propinyloxy, Butinyloxy, Propenylthio, Butenylthio, Propinylthio, Butinylthio, Propenylamino, Butenylamino, Propinylamino oder Butinylamino, für Dimethylamino, Diethylamino oder Dipropylamino, für jeweils gegebenenfalls durch Fluor, Chlor, Methyl und/oder Ethyl substituiertes Cyclopropyl, Cyclobutyl, Cyclopentyl, Cyclohexyl, Cyclopentenyl, Cyclohexenyl, Cyclopropyloxy, Cyclobutyloxy, Cyclopentyloxy, Cyclohexyloxy, Cyclopropylthio, Cyclobutylthio, Cyclopentylthio, Cyclohexylthio, Cyclopropylamino, Cyclobutylamino, Cyclopentylamino, Cyclohexylamino, Cyclopropylmethyl, Cyclobutylmethyl, Cyclopentylmethyl, Cyclohexylmethyl, Cyclopropylmethoxy, Cyclobutylmethoxy, Cyclopentylmethoxy, Cyclohexylmethoxy, Cyclopropylmethylthio, Cyclobutylmethylthio, Cyclopentylmethylthio, Cyclohexylmethylthio, Cyclopropylmethylamino, Cyclobutylmethylamino, Cyclopentylmethylamino oder Cyclohexylmethylamino, oder für jeweils gegebenenfalls durch Fluor, Chlor, Brom, Methyl, Trifluormethyl, Methoxy oder Methoxy-carbonyl substituiertes Phenyl, Benzyl, Phenoxy, Benzyloxy, Phenylthio, Benzylthio, Phenylamino oder Benzylamino.
- R⁴: steht bevorzugt für Wasserstoff, Hydroxy, Amino, für jeweils gegebenenfalls durch Fluor, Chlor, Cyano, Methoxy oder Ethoxy substituiertes Methyl, Ethyl, n- oder i-Propyl, n-, i-, s- oder t-Butyl, für jeweils gegebenenfalls durch Fluor, Chlor und/oder Brom substituiertes Ethenyl, Propenyl, Butenyl, Propinyl oder Butinyl, für jeweils gegebenenfalls durch Fluor, Chlor, Cyano, Methoxy oder Ethoxy substituiertes Methoxy, Ethoxy, n- oder i-Propoxy, n-, i-, s- oder t-Butoxy, Methylamino, Ethylamino, n- oder i-Propylamino, n-, i-, s- oder t-Butylamino, für Propenyloxy oder Butenyloxy, für Dimethylamino oder Diethylamino, für jeweils gegebenenfalls durch Fluor, Chlor, Methyl und/oder Ethyl substituiertes Cyclopropyl, Cyclobutyl, Cyclopentyl, Cyclohexyl, Cyclopropylamino, Cyclobutylamino, Cyclopentylamino, Cyclohexylamino, Cyclopropylmethyl, Cyclobutylmethyl, Cyclopentylmethyl oder Cyclohexylmethyl, oder für jeweils gegebenenfalls durch Fluor, Chlor, Methyl, Trifluormethyl und/oder Methoxy substituiertes Phenyl oder Benzyl.
- R³ und R⁴: zusammen stehen auch bevorzugt zusammen für Trimethylen (Propan-1,3-diyl), Tetramethylen (Butan-1,4-diyl) oder Pentamethylen (Pentan-1,5-diyl).
- Q¹: steht besonders bevorzugt für O (Sauerstoff).
- Q²: steht besonders bevorzugt für O (Sauerstoff).
- R¹: steht besonders bevorzugt für jeweils gegebenenfalls durch Fluor, Chlor, Methoxy oder Ethoxy substituiertes Methyl, Ethyl, n- oder i-Propyl,
- R²: steht besonders bevorzugt für Fluor, Chlor, Brom oder für jeweils gegebenenfalls durch Fluor, Chlor, Methoxy oder Ethoxy substituiertes Methyl, Ethyl, n- oder i-Propyl.
- R³: steht besonders bevorzugt für Wasserstoff, Chlor, Brom, für jeweils gegebenenfalls durch Fluor, Chlor, Methoxy, Ethoxy, n- oder i-Propoxy substituiertes Methyl, Ethyl, n- oder i-Propyl, für jeweils gegebenenfalls durch Fluor oder Chlor substituiertes Ethenyl, Propenyl, Butenyl, Propinyl oder Butinyl, für jeweils gegebenenfalls durch Fluor, Chlor, Methoxy, Ethoxy, n- oder i-Propoxy substituiertes Methoxy, Ethoxy, n- oder i-Propoxy, Methylthio, Ethylthio, n- oder i-Propylthio, Methylamino, Ethylamino, n- oder i-Propylamino, für Propenyloxy, Propinyloxy, Propenylthio, Propinylthio, Propenylamino oder Propinylamino, für Dimethylamino oder Diethylamino, für jeweils gegebenenfalls durch Fluor, Chlor oder Methyl substituiertes Cyclopropyl, Cyclopropyloxy, Cyclopropylmethyl oder Cyclopropylmethoxy.
- R⁴: steht besonders bevorzugt für jeweils gegebenenfalls durch Fluor, Chlor, Methoxy oder Ethoxy substituiertes Methyl, Ethyl, n- oder i-Propyl, für jeweils gegebenenfalls durch Fluor oder Chlor substituiertes Ethenyl, Propenyl oder Propinyl, für jeweils gegebenenfalls durch Fluor, Chlor, Methoxy oder Ethoxy substituiertes Methoxy, Ethoxy, n- oder i-Propoxy, für Methylamino, oder für Cyclopropyl.
- R¹: steht am meisten bevorzugt für Methyl, Ethyl, n- oder i-Propyl.
- R²: steht am meisten bevorzugt für Methyl, Ethyl, n- oder i-Propyl.
- R³: steht am meisten bevorzugt für Methoxy, Ethoxy, n- oder i-Propoxy, Methyl, Ethyl, n- oder 1-Propyl, Methylthio, Ethylthio, n- oder i-Propylthio oder Cyclopropyl.
- R⁴: steht am meisten bevorzugt für Methyl, Ethyl, n- oder i-Propyl oder Cyclopropyl.

Gegenstand der Erfindung sind vorzugsweise auch die Natrium-, Kalium-, Magnesium-, Calcium-, Ammonium-, C₁-C₄-Alkyl-ammonium-, Di-(C₁-C₄-alkyl)-ammonium-, Tri-(C₁-C₄-alkyl)-ammonium-, Tetra-(C₁-C₄-alkyl)-ammonium, Tri-(C₁-C₄-alkyl)-sulfonium-, C₅- oder C₆-Cycloalkyl-ammonium- und Di-(C₁-C₂alkyl)-benzyl-ammonium-Salze von Verbindungen der Formel (I), in welcher Q¹, Q², R¹, R², R³ und R⁴ die oben vorzugsweise angegebenen Bedeutungen haben.

Die oben aufgeführten allgemeinen oder in Vorzugsbereichen aufgeführten Restedefinitionen gelten sowohl für die Endprodukte der Formel (I) als auch entsprechend für die jeweils zur Herstellung benötigten Ausgangs- oder Zwischenprodukte. Diese Restedefinitionen können untereinander, also auch zwischen den angegebenen bevorzugten Bereichen beliebig kombiniert werden.

Erfindungsgemäß bevorzugt sind diejenigen Verbindungen der Formel (I), bei welchen eine Kombination der vorstehend als bevorzugt aufgeführten Bedeutungen vorliegt.

Erfindungsgemäß besonders bevorzugt sind diejenigen Verbindungen der Formel (I), bei welchen eine Kombination der vorstehend als besonders bevorzugt aufgeführten Bedeutungen vorliegt.

Erfindungsgemäß ganz besonders bevorzugt sind diejenigen Verbindungen der Formel (I), bei welchen eine Kombination der vorstehend als ganz besonders bevorzugt aufgeführten Bedeutungen vorliegt.

Die neuen substituierten Thien-3-yl-sulfonylamino(thio)carbonyl-triazolin(thi)one der allgemeinen Formel (I) weisen interessante biologische Eigenschaften auf. Sie zeichnen sich insbesondere durch starke herbizide Wirksamkeit aus.

Man erhält die neuen substituierten Thien-3-yl-sulfonylamino(thio)carbonyltriazolin(thi)one der allgemeinen Formel (I), wenn man
(a) substituierte Thiophen-3-sulfonamide der allgemeinen Formel (II) 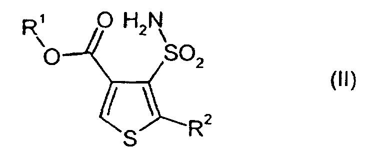 in welcher
   - R¹ und R²: die oben angegebene Bedeutung haben,
   mit substituierten Triazolin(thi)onen der allgemeinen Formel (III) 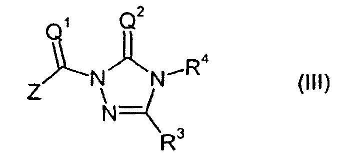 in welcher
   - Q¹, Q², R³ und R⁴: die oben angegebene Bedeutung haben und
   - Z: für Halogen, Alkoxy, Aryloxy oder Arylalkoxy steht,
   gegebenenfalls in Gegenwart eines Reaktionshilfsmittels und gegebenenfalls in Gegenwart eines Verdünnungsmittels umsetzt,
   oder wenn man
(b) substituierte Thien-3-yl-sulfonyl-iso(thio)cyanate der allgemeinen Formel (IV) 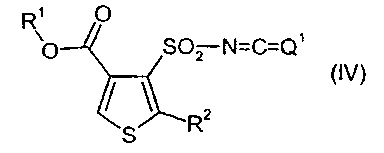 in welcher
   - Q¹, R¹ und R²: die oben angegebene Bedeutung haben,
   mit Triazolin(thi)onen der allgemeinen Formel (V) 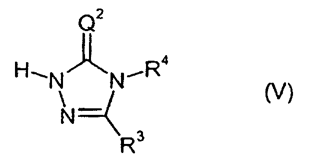 in welcher
   - Q², R⁴ und R⁵: die oben angegebene Bedeutung haben,
   gegebenenfalls in Gegenwart eines Reaktionshilfsmittels und gegebenenfalls in Gegenwart eines Verdünnungsmittels umsetzt,
   oder wenn man
(c) substituierte Thiophen-3-sulfonsäurechloride der allgemeinen Formel (VI) 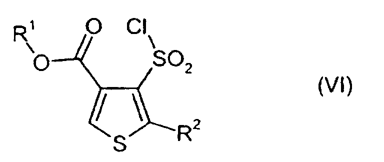 in welcher
   - R¹ und R²: die oben angegebene Bedeutung haben,
   mit Triazolin(thi)onen der allgemeinen Formel (V) 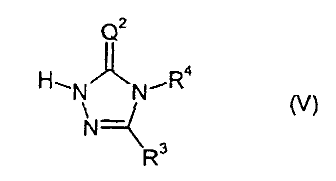 in welcher
   - Q², R⁴ und R⁵: die oben angegebene Bedeutung haben,
   und Metall(thio)cyanaten der allgemeinen Formel (VII)

   M-Q¹-CN (VII)

   in welcher
   - Q¹: die oben angegebene Bedeutung hat,
   gegebenenfalls in Gegenwart eines Reaktionshilfsmittels und gegebenenfalls in Gegenwart eines Verdünnungsmittels umsetzt,
   oder wenn man
(d) substituierte Thiophen-3-sulfonsäurechloride der allgemeinen Formel (VI) 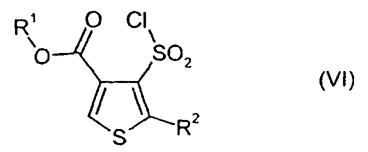 in welcher
   - R¹ und R²: die oben angegebene Bedeutung haben,
   mit Triazolin(thi)on-(thio)carboxamiden der allgemeinen Formel (VIII) 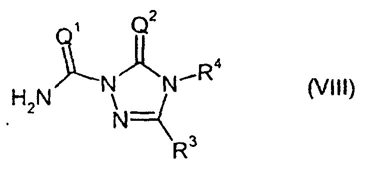 in welcher
   - Q¹, Q², R³ und R⁴: die oben angegebene Bedeutung haben,
   gegebenenfalls in Gegenwart eines Reaktionshilfsmittels und gegebenenfalls in Gegenwart eines Verdünnungsmittels umsetzt,
   oder wenn man
(e) substituierte Thien-3-yl-sulfonylamino(thio)carbonylverbindungen der allgemeinen Formel (IX) 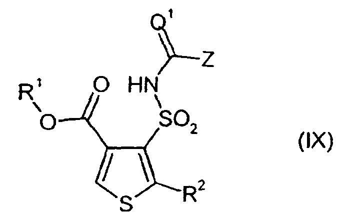 in welcher
   - Q¹, R¹ und R²: die oben angegebene Bedeutung haben und
   - Z: für Halogen, Alkoxy, Aryloxy oder Arylalkoxy steht,
   mit Triazolin(thi)onen der allgemeinen Formel (V) 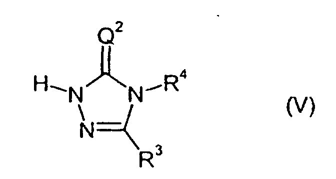 in weicher
   - Q², R⁴ und R⁵: die oben angegebene Bedeutung haben,
   gegebenenfalls in Gegenwart eines Reaktionshilfsmittels und gegebenenfalls in Gegenwart eines Verdünnungsmittels umsetzt,
und gegebenenfalls die nach den Verfahren (a), (b), (c), (d) oder (e) erhaltenen Verbindungen der Formel (I) nach üblichem Methoden in Salze überführt.

Verwendet man beispielsweise 2-Chlor-4-ethoxycarbonyl-thiophen-3-sulfonamid und 4,5-Dimethoxy-2-phenoxycarbonyl-2,4-dihydro-3H-1,2,4-triazol-3-on als Ausgangsstoffe, so kann der Reaktionsablauf beim erfindungsgemäßen Verfahren (a) durch das folgende Formelschema skizziert werden:

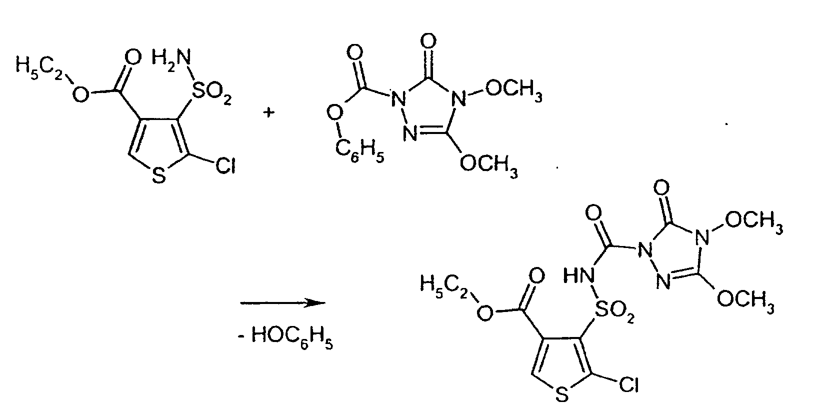

Verwendet man beispielsweise 2-Fluor-4-methoxycarbonyl-thien-3-yl-sulfonyl-isothiocyanat und 5-Ethoxy-4-methyl-2,4-dihydro-3H-1,2,4-triazol-3-on als Ausgangsstoffe, so kann der Reaktionsablauf beim erfindungsgemäßen Verfahren (b) durch das folgende Formelschema skizziert werden:

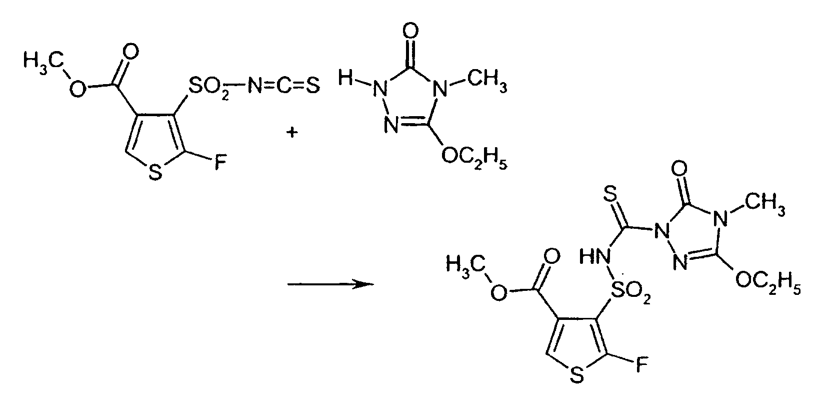

Verwendet man beispielsweise 4-Methoxycarbonyl-2-trifluormethyl-thiophen-3-sulfonsäurechlorid, 5-Ethyl-4-methoxy-2,4-dihydro-3H-1,2,4-triazol-3-thion und Kaliumcyanat als Ausgangsstoffe, so kann der Realktionsablauf beim erfindungsgemäßen Verfahren (c) durch das folgende Formelschema skizziert werden:

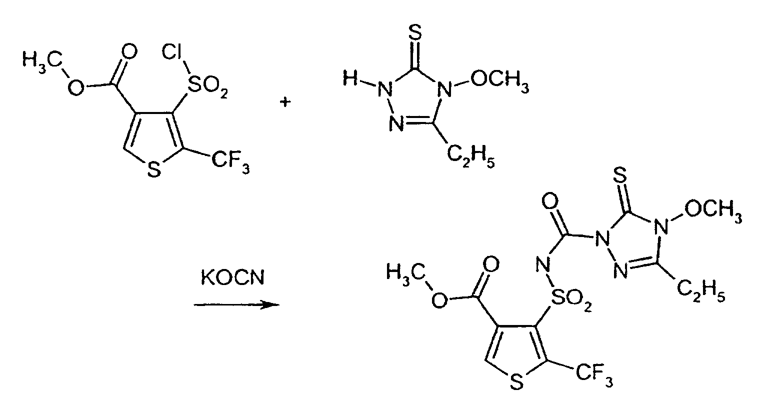

Verwendet man beispielsweise 3-Ethoxycarbonyl-2-methyl-thiophen-4-sulfonsäurechlorid und 4-Ethyl-5-methoxy-2,4-dihydro-3H-1,2,4-triazol-3-on-2-carboxamid als Ausgangsstoffe, so kann der Reaktionsablauf beim erfindungsgemäßen Verfahren (d) durch das folgende Formelschema skizziert werden:

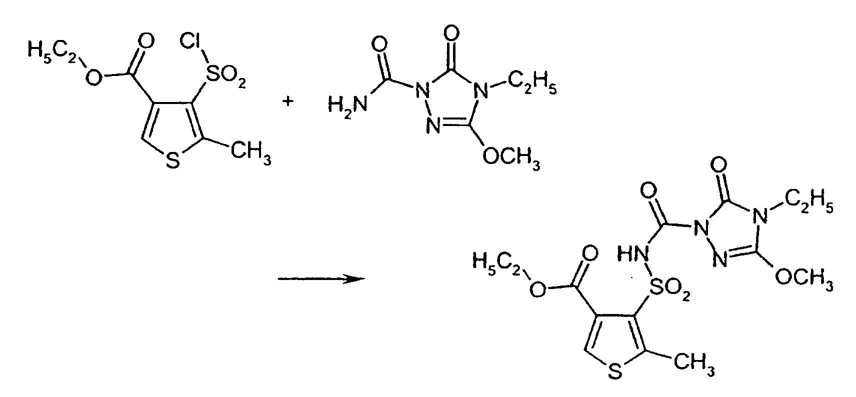

Verwendet man beispielsweise N-(2-Ethyl-4-i-propoxycarbonyl-thien-3-yl-sulfonyl)-O-methyl-urethan und 4,5-Dimethyl-2,4-dihydro-3H-1,2,4-triazol-3-on als Ausgangsstoffe, so kann der Reaktionsablauf beim erfindungsgemäßen Verfahren (e) durch das folgende Formelschema skizziert werden:

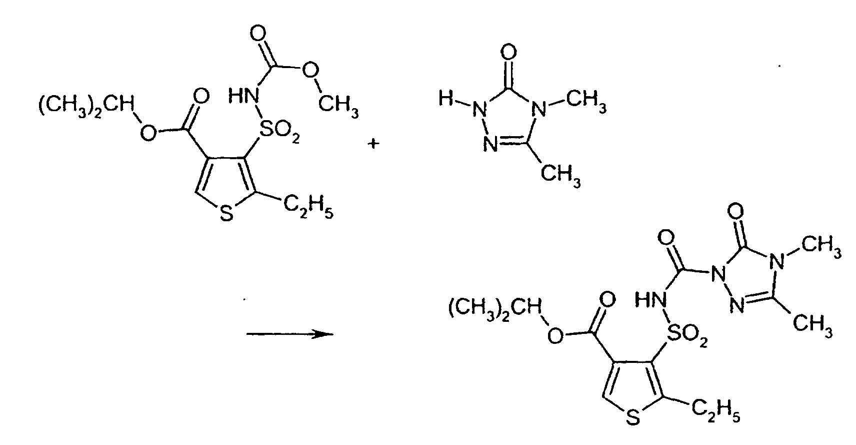

Die beim erfindungsgemäßen Verfahren (a) zur Herstellung von Verbindungen der allgemeinen Formel (1) als Ausgangsstoffe zu verwendenden substituierten Thiophen-3-sulfonamide sind durch die Formel (II) allgemein definiert. In der allgemeinen Formel (II) haben R¹ und R² vorzugsweise diejenigen Bedeutungen, die bereits oben im Zusammenhang mit der Beschreibung der erfindungsgemäßen Verbindungen der allgemeinen Formel (I) vorzugsweise bzw. in einer der besonders bevorzugten Definitionen für R¹ und R² angegeben worden sind.

Die substituierten Thiophen-3-sulfonamide der allgemeinen Formel (II) sind mit Ausnahme von 4-Methoxycarbonyl-thiophen-3-sulfonamid (vgl. J. Org. Chem. 45 (1980), 617-620) noch nicht aus der Literatur bekannt; sie sind unter Ausnahme von 4-Methoxycarbonyl-thiophen-3-sulfonamid als neue Stoffe auch Gegenstand der vorliegenden Anmeldung.

Man erhält die substituierten Thiophen-3-sulfonamide der allgemeinen Formel (II), wenn man substituierte Thiophen-3-sulfonsäurechloride der allgemeinen Formel (VI) 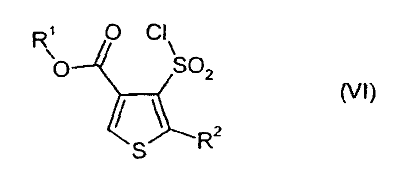 in welcher
- R¹ und R²: die oben angegebene Bedeutung haben,
mit Ammoniak oder mit Ammoniumsalzen, wie z.B. Ammoniumacetat oder Ammoniumcarbonat, gegebenenfalls in Gegenwart eines Verdünnungsmittels, wie z.B. Wasser oder Methylenchlorid, bei Temperaturen zwischen 0°C und 100°C umsetzt (vgl. die Herstellungsbeispiele).

Die beim erfindungsgemäßen Verfahren (a) zur Herstellung von Verbindungen der allgemeinen Formel (I) weiter als Ausgangsstoffe zu verwendenden substituierten Triazolin(thi)one sind durch die Formel (III) allgemein definiert. In der allgemeinen Formel (III) haben Q¹, Q², R³ und R⁴ vorzugsweise diejenigen Bedeutungen, die bereits oben im Zusammenhang mit der Beschreibung der erfindungsgemäßen Verbindungen der allgemeinen Formel (I) vorzugsweise bzw. in einer der besonders bevorzugten Definitionen für Q¹, Q², R³ und R⁴ angegeben worden sind.

Die Ausgangsstoffe der allgemeinen Formel (III) sind bekannt und/oder können nach an sich bekannten Verfahren hergestellt werden (vgl. EP-A-341 489, EP-A-422 469, EP-A-425 948, EP-A-431 291, EP-A-507 171, EP-A-534 266).

Die beim erfindungsgemäßen Verfahren (b) zur Herstellung von Verbindungen der allgemeinen Formel (I) als Ausgangsstoffe zu verwendenden substituierten Thien-3-yl-sulfonyl-iso(thio)cyanate sind durch die Formel (IV) allgemein definiert. In der allgemeinen Formel (IV) haben Q¹, R¹ und R² vorzugsweise diejenigen Bedeutungen, die bereits oben im Zusammenhang mit der Beschreibung der erfindungsgemäßen Verbindungen der allgemeinen Formel (I) vorzugsweise bzw. in einer der besonders bevorzugten Definitionen für Q¹, R¹ und R² angegeben worden sind.

Die Ausgangsstoffe der allgemeinen Formel (IV) sind bekannt und/oder können nach an sich bekannten Verfahren hergestellt werden (vgl. US-A-47 01 535).

Die bei den erfindungsgemäßen Verfahren (b), (c) und (e) zur Herstellung von Verbindungen der allgemeinen Formel (I) als Ausgangsstoffe zu verwendenden Triazolin(thi)one sind durch die Formel (V) allgemein definiert. In der allgemeinen Formel (V) haben Q², R⁴ und R⁵ vorzugsweise diejenigen Bedeutungen, die bereits oben im Zusammenhang mit der Beschreibung der erfindungsgemäßen Verbindungen der allgemeinen Formel (I) vorzugsweise bzw. in einer der besonders bevorzugten Definitionen für Q², R⁴und R⁵ angegeben worden sind.

Die Ausgangsstoffe der allgemeinen Formel (V) sind bekannt und/oder können nach an sich bekannten Verfahren hergestellt werden (vgl. EP-A-341 489, EP-A-422 469, EP-A-425 948, EP-A-431 291, EP-A-507 171, EP-A-534 266).

Die bei den erfindungsgemäßen Verfahren (c) und (d) zur Herstellung von Verbindungen der allgemeinen Formel (I) als Ausgangsstoffe zu verwendenden substituierten Thiophen-3-sulfonsäurechloride sind durch die Formel (VI) allgemein definiert. In der allgemeinen Formel (VI) haben R¹ und R² vorzugsweise diejenigen Bedeutungen, die bereits oben im Zusammenhang mit der Beschreibung der erfindungsgemäßen Verbindungen der allgemeinen Formel (I) vorzugsweise, bzw. in einer der besonders bevorzugten Definitionen für R¹ und R² angegeben worden sind.

Die substituierten Thiophen-3-sulfonsäurechloride der allgemeinen Formel (VI) sind zum Teil (vgl. J. Org. Chem. 45 (1980), 617-620 und EP 1113) aus der Literatur bekannt; sie sind unter Ausnahme von 4-Methoxycarbonyl-thiophen-3-sulfonsäurechlorid als neue Stoffe auch Gegenstand der vorliegenden Anmeldung, wenn R² die oben angegebene Bedeutung hat, aber nicht für Halogen steht.

Man erhält die substituierten Thiophen-3-sulfonsäurechloride der allgemeinen Formel (VI), wenn man 3-Amino-thiophen-4-carbonsäureester der allgemeinen Formel (X) 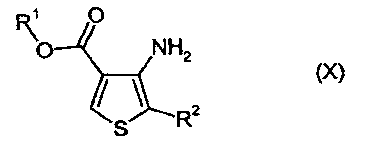 in welcher
- R¹ und R²: die oben angegebene Bedeutung haben,
- oder Säureaddukte von Verbindungen der Formel (X), wie z.B. die Hydrochloride -
mit einem Alkalimetallnitrit. wie z.B. Natriumnitrit, in Gegenwart von Salzsäure bei Temperaturen zwischen -10°C und +10°C umsetzt und die so erhaltene Diazoniumsalzlösung mit Schwefeldioxid in Gegenwart eines Verdünnungsmittels, wie z.B. Dichlormethan, 1,2-Dichlor-ethan oder Essigsäure, und in Gegenwart eines Katalysators, wie z.B. Kupfer(I)-chlorid und/oder Kupfer(II)-chlorid, bei Temperaturen zwischen -10°C und +50°C umsetzt.

Die Vorprodukte der allgemeinen Formel (X) sind bekannt und/oder können nach an sich bekannten Verfahren hergestellt werden (vgl. Austr. J. Chem. 48 (1995), 1907-1916; Herstellungsbeispiele).

Die beim erfindungsgemäßen Verfahren (d) zur Herstellung von Verbindungen der allgemeinen Formel (I) als Ausgangsstoffe zu verwendenden Triazolin(thi)on-(thio)-carboxamide sind durch die Formel (VIII) allgemein definiert. In der allgemeinen Formel (VIII) haben Q¹, Q², R³ und R⁴ vorzugsweise diejenigen Bedeutungen, die bereits oben im Zusammenhang mit der Beschreibung der erfindungsgemäßen Verbindungen der allgemeinen Formel (I) vorzugsweise bzw. in einer der besonders bevorzugten Definitionen für Q¹, Q², R³ und R⁴ angegeben worden sind.

Die Ausgangsstoffe der allgemeinen Formel (VIII) sind bekannt und/oder können nach an sich bekannten Verfahren hergestellt werden.

Die beim erfindungsgemäßen Verfahren (e) zur Herstellung von Verbindungen der allgemeinen Formel (I) als Ausgangsstoffe zu verwendenden substituierten Thien-3-yl-sulfonylamino(thio)carbonylverbindungen sind durch die Formel (IX) allgemein definiert. In der allgemeinen Formel (IX) haben Q¹, R¹ und R² vorzugsweise diejenigen Bedeutungen, die bereits oben im Zusammenhang mit der Beschreibung der erfindungsgemäßen Verbindungen der allgemeinen Formel (I) vorzugsweise bzw. in einer der besonders bevorzugten Definitionen für Q¹, R¹ und R² angegeben worden sind.

Die Ausgangsstoffe der allgemeinen Formel (IX) sind bekannt und/oder können nach an sich bekannten Verfahren hergestellt werden.

Die erfindungsgemäßen Verfahren (a), (b), (c), (d) und (e) zur Herstellung der neuen Verbindungen der Formel (I) werden vorzugsweise unter Verwendung von Verdünnungsmitteln durchgeführt. Als Verdünnungsmittel kommen dabei praktisch alle inerten organischen Lösungsmittel infrage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlormethan, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methylethyl-, Methyl-isopropyl- und Methyl-isobutyl-keton, Ester wie Essigssäuremethylester und -ethylester, Nitrite wie z.B. Acetonitril und Propionitril, Amide wie z.B. Dimethylformamid, Dimethylacetamid und N-Methylpyrrolidon sowie Dimethylsulfoxid, Tetramethylensulfon und Hexamethylphosphorsäuretriamid.

Als Reaktionshilfsmittel können bei den erfindungsgemäßen Verfahren (a), (b), (c), (d) und (e) alle üblicherweise für derartige Umsetzungen verwendbaren Säurebindemittel eingesetzt werden. Vorzugsweise infrage kommen Alkalimetallhydroxide wie z.B. Natrium- und Kaliumhydroxid, Erdalkalihydroxide wie z.B. Calciumhydroxid, Alkalicarbonate und -alkoholate wie Natrium- und Kaliumcarbonat, Natrium- und Kalium-tert-butylat, ferner basische Stickstoffverbindungen, wie Trimethylamin, Triethylamin, Tripropylamin, Tributylamin, Diisobutylamin, Dicyclohexylamin, Ethyldiisopropylamin, Ethyldicyclohexylamin, N,N-Dimethylbenzylamin, -N,N-Dimethyl-anilin, Pyridin, 2-Methyl-, 3-Methyl-, 4-Methyl-, 2,4-Dimethyl-, 2,6-Dimethyl-, 2-Ethyl-, 4-Ethyl- und 5-Ethyl-2-methyl-pyridin, 1,5-Diazabicyclo[4.3.0]-non-5-en (DBN), 1,8-Diazabicyclo-[5.4.0]-undec-7-en (DBU) und 1,4-Diazabicyclo-[2.2.2]-octan (DABCO).

Die Reaktionstemperaturen können bei den erfindungsgemäßen Verfahren (a), (b), (c), (d) und (e) in einem größeren Bereich variiert werden. Im Allgemeinen arbeitet man bei Temperaturen zwischen -20°C und +150°C, vorzugsweise bei Temperaturen zwischen 0°C und +100°C.

Die erfindungsgemäßen Verfahren (a), (b), (c), (d) und (e) werden im Allgemeinen unter Normaldruck durchgeführt. Es ist jedoch auch möglich, unter erhöhtem oder vermindertem Druck zu arbeiten.

Zur Durchführung der erfindungsgemäßen Verfahren (a), (b), (c), (d) und (e) werden die jeweils benötigten Ausgangsstoffe im Allgemeinen in angenähert äquimolaren Mengen eingesetzt. Es ist jedoch auch möglich, eine der jeweils eingesetzten Komponenten in einem größeren Überschuß zu verwenden. Die Reaktionen werden im Allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt bei den erfindungsgemäßen Verfahren (a), (b), (c), (d) und (e) jeweils nach üblichen Methoden (vgl. die Herstellungsbeispiele).

Aus den erfindungsgemäßen Verbindungen der allgemeinen Formel (I) können gegebenenfalls Salze hergestellt werden. Man erhält solche Salze in einfacher Weise nach üblichen Salzbildungsmethoden, beispielsweise durch Lösen oder Dispergieren einer Verbindung der Formel (I) in einem geeigneten Lösungsmittel, wie z.B. Methylenchlorid, Aceton, tert-Butyl-methylether oder Toluol, und Zugabe einer geeigneten Base. Die Salze können dann - gegebenenfalls nach längerem Rühren - durch Einengen oder Absaugen isoliert werden.

Die erfindungsgemäßen Wirkstoffe können als Defoliants, Desiccants, Krautabtötungsmittel und insbesondere als Unkrautvemichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im Wesentlichen von der angewendeten Menge ab.

Die erfindungsgemäßen Wirkstoffe können z.B. bei den folgenden Pflanzen verwendet werden:
Dikotyle Unkräuter der Gattungen: Abutilon, Amaranthus, Ambrosia, Anoda, Anthemis, Aphanes, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.
Dikotyle Kulturen der Gattungen: Arachis, Beta, Brassica, Cucumis, Cucurbita, Helianthus, Daucus, Glycine, Gossypium, lpomoea, Lactuca, Linum, Lycopersicon, Nicotiana, Phaseolus, Pisum, Solanum, Vicia.
Monokotyle Unkräuter der Gattungen: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.
Monokotyle Kulturen der Gattungen: Allium, Ananas, Asparagus, Avena, Hordeum, Oryza, Panicum, Saccharum, Secale, Sorghum, Triticale, Triticum, Zea.

Die Verwendung der erfindungsgemäßen Wirkstoffe ist jedoch keineswegs auf diese Gattungen beschränkt, sondern erstreckt sich in gleicher Weise auch auf andere Pflanzen.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Spross, Blatt, Blüte und Wurzel verstanden werden, wobei beispielhaft Blätter, Nadeln, Stengel, Stämme, Blüten, Fruchtkörper, Früchte und Samen sowie Wurzeln, vegetatives und generatives Vennehrungsmaterial, beispielsweise Stecklinge, Knollen, Rhizome, Ableger und Samen.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen und bei Vermehrungsmaterial, insbesondere bei Samen, weiterhin durch ein- oder mehrschichtiges Umhüllen.

Die erfindungsgemäßen Wirkstoffe eignen sich in Abhängigkeit von der Konzentration zur Totalunkrautbekämpfung, z.B. auf Industrie- und Gleisanlagen und auf Wegen und Plätzen mit und ohne Baumbewuchs. Ebenso können die erfindungsgemäßen Wirkstoffe zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen sowie zur selektiven Unkrautbekämpfung in einjährigen Kulturen eingesetzt werden.

Die erfindungsgemäßen Verbindungen der Formel (I) zeigen starke herbizide Wirksamkeit und ein breites Wirkungsspektrum bei Anwendung auf dem Boden und auf oberirdische Pflanzenteile. Sie eignen sich in gewissem Umfang auch zur selektiven Bekämpfung von monokotylen und dikotylen Unkräutern in monokotylen und dikotylen Kulturen, sowohl im Vorauflauf- als auch im Nachauflauf-Verfahren.

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im Wesentlichen infrage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen infrage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen infrage: z.B. nichtionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen infrage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im Allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide infrage, beispielsweise
Acetochlor, Acifluorfen(-sodium), Aclonifen, Alachlor, Alloxydim(-sodium), Ametryne, Amidochlor, Amidosulfuron, Anilofos, Asulam, Atrazine, Azafenidin, Azimsulfuron, Benazolin(-ethyl), Benfurcsate, Bensulfuron(-methyl), Bentazon, Benzobicyclon, Benzofenap, Benzoylprop(-ethyl), Bialaphos, Bifenox, Bispyribac(sodium), Bromobutide, Bromofenoxim, Bromoxynil, Butachlor, Butroxydim, Butylate, Cafenstrole, Caloxydim, Carbetamide, Carfentrazone(-ethyl), Chlomethoxyfen, Chloramben, Chloridazon, Chlorimuron(-ethyl), Chlomitrofen, Chlorsulfuron, Chlortoluron, Cinidon(-ethyl), Cinmethylin, Cinosulfuron, Clefoxydim, Clethodim, Clodinafop(-propargyl), Clomazone, Clomeprop, Clopyralid, Clopyrasulfuron(-methyl), Cloransulam(-methyl), Cumyluron, Cyanazine, Cybutryne, Cycloate, Cyclosulfamuron, Cycloxydim, Cyhalofop(-butyl), 2,4-D, 2,4-DB, 2,4-DP, Desmedipham, Diallate, Dicamba, Diclofop(-methyl), Diclosulam, Diethatyl(-ethyl), Difenzoquat, Diflufenican, Diflufenzopyr, Dimefuron, Dimepiperate, Dimethachlor, Dimethametryn, Dimethenamid, Dimexyflam, Dinitramine, Diphenamid, Diquat, Dithiopyr, Diuron, Dymron, Epropodan, EPTC, Esprocarb, Ethalfluralin, Ethametsulfuron(-methyl), Ethofumesate, Ethoxyfen, Ethoxysulfuron, Etobenzanid, Fenoxaprop(-P-ethyl), Fentrazamide, Flamprop(-isopropyl), Flamprop(-isopropyl-L), Flamprop(methyl), Flazasulfuron, Florasulam, Fluazifop(-P-butyl), Fluazolate, Flucarbazone, Flufenacet, Flumetsulam, Flumiclorac(-pentyl), Flumioxazin, Flumipropyn, Flumetsulam, Fluometuron, Fluorochloridone, Fluoroglycofen(-ethyl), Flupoxam, Flupropacil, Flurpyrsulfuron(-methyl, -sodium), Flurenol(-butyl), Fluridone, Fluroxypyr(meptyl), Flurprimidol, Flurtamone, Fluthiacet(-methyl), Fluthiamide, Fomesafen, Glufosinate(-ammonium), Glyphosate(-isopropylammonium), Halosafen, Haloxyfop(-ethoxyethyl), Haloxyfop(-P-methyl), Hexazinone, Imazamethabenz(-methyl), Imazamethapyr, Imazamox, Imazapic, Imazapyr, Imazaquin, Imazethapyr, Imazösulfuron, lodosulfuron(-methyl, -sodium), Ioxynil, Isopropalin, Isoproturon, Isouron, Isoxaben, Isoxachlortole, Isoxaflutole, Isoxapyrifop, Lactofen, Lenacil, Linuron, MCPA, MCPP, Mefenacet, Mesotrione, Metamitron, Metazachlor, Methabenzthiazuron, Metobenzuron, Metobromuron, (alpha-)Metolachlor, Metosulam, Metoxuron, Metribuzin, Metsulfuron(-methyl), Molinate, Monolinuron, Naproanilide, Napropamide, Neburon, Nicosulfuron, Norflurazon, Orbencarb, Oryzalin, Oxadiargyl, Oxadiazon, Oxasulfuron, Oxaziclomefone, Oxyfluorfen, Paraquat, Pelargonsäure, Pendimethalin, Pendralin, Pentoxazone, Phenmedipham, Piperophos, Pretilachlor, Primisalfuron(-methyl), Prometryn, Propachlor, Propanil, Propaquizafop, Propisochlor, Propyzamide, Prosulfocarb, Prosulfuron, Pyraflufen(-ethyl), Pyrazolate, Pyrazosulfuron(-ethyl), Pyrazoxyfen, Pyribenzoxim, Pyributicarb, Pyridate, Pyriminobac(methyl), Pyrithiobac(-sodium), Quinchlorac, Quinmerac, Quinoclamine, Quizalofop(-P-ethyl), Quizalofop(-P-tefuryl), Rimsulfuron, Sethoxydim, Simazinc, Simetryn, Sulcotrione, Sulfentrazone, Sulfometuron(-methyl), Sulfosate, Sulfosulfuron, Tebutam, Tebuthiuron, Tepraloxydim, Terbuthylazine, Terbutryn, Thenylchlor, Thiafluamide, Thiazopyr, Thidiazimin, Thifensulfuron(-methyl), Thiobencarb, Tiocarbazil, Tralkoxydim, Triallate, Triasulfuron, Tribenuron(-methyl), Triclopyr, Tridiphane, Trifluralin und Triflusulfuron.

Auch eine Mischung mit anderen bekannten Wirkstoffen, wie Fungiziden, Insektiziden, Akariziden, Nematiziden, Schutzstoffen gegen VogelfraB, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im Wesentlichen von der Art des gewünschten Effektes ab. Im Allgemeinen liegen die Aufwandmengen zwischen 1 g und 10 kg Wirkstoff pro Hektar Bodenfläche, vorzugsweise zwischen 5 g und 5 kg pro ha.

Die Herstellung und die Verwendung der erfindungsgemäßen Wirkstoffe geht aus den nachfolgenden Beispielen hervor.

### Herstellungsbeispiele:

### Beispiel 1

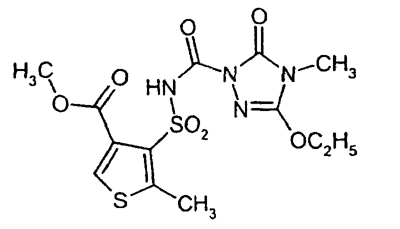

### (Verfahren (a))

0,76 g (2,9 mMol) S-Ethoxy-4-methyl-2-phenoxycarbonyl-2,4-dihydro-3H-1,2,4-triazol-3-on werden in 40 ml Acetonitril gelöst und bei Raumtemperatur (ca. 20°C) unter Rühren portionsweise mit 0,75 g (3,2 mMol) 4-Methoxycarbonyl-2-methylthiophen-3-sulfonamid und 0,49 g (3,2 mMol) 1,8-Diazabicyclo-[5.4.0]-undec-7-en (DBU) versetzt. Die Reaktionsmischung wird 12 Stunden bei Raumtemperatur gerührt und anschließend unter vermindertem Druck eingeengt. Der Rückstand wird in Methylenchlorid aufgenommen, nacheinander mit 1N-Salzsäure und mit Wasser gewaschen, mit Natriumsulfat getrocknet und filtriert. Das Filtrat wird im Wasserstrahlvakuum eingeengt, der Rückstand mit Isopropanol digeriert und das kristallin angefallene Produkt durch Absaugen isoliert.

Man erhält 0,70 g (60% der Theorie) 4-[[((3-Ethoxy-4,5-dihydro-4-methyl-5-oxo-1H-1,2,4-triazol-1-yl)-carbonyl]-amino]-sulfonyl]-5-methyl-thiophen-3-carbonsäuremethylester (alias 5-Ethoxy-4-methyl-2-[(4-methoxycarbonyl-2-methyl-thien-3-yl)-sulfonyl-amino-carbonyl]-2,4-dihydro-3H-1,2,4-triazol-3-on) vom Schmelzpunkt 163°C.

Analog zu Beispiel 1 sowie entsprechend dcr allgemeinen Beschreibung der erfindungsgemäßen Herstellungsverfahren können beispielsweise auch die in der nachstehenden Tabelle 1 aufgeführten Verbindungen der allgemeinen Formel (I) hergestellt werden.

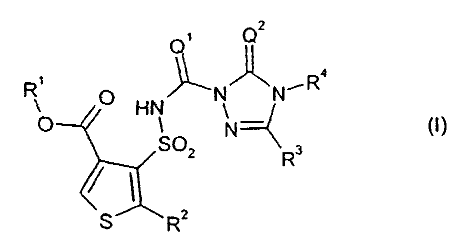

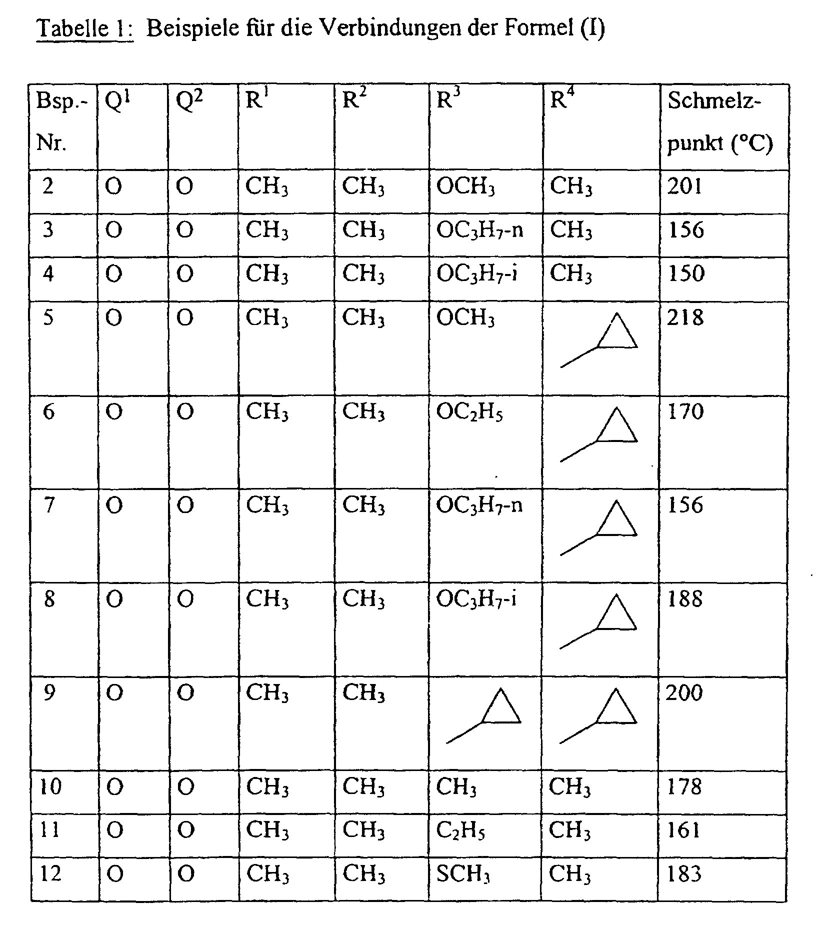

### Ausgangsstoffe der Formel (II):

### Beispiel (II-1)

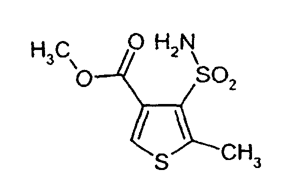

Eine Mischung aus 45 g (177 mMol) 4-Methoxycarbonyl-2-methyl-thiophen-3-sulfonsäurechlorid, 34 g (354 mMol) Ammoniumcarbonat und 400 ml Methylenchlorid wird 12 Stunden bei Raumtemperatur (ca. 20°C) gerührt. Nach Filtration wird vom Filtrat das Lösungsmittel im Wasserstrahlvakuum abdestilliert, der Rückstand mit Diethylether digeriert und das kristalline Produkt durch Absaugen isoliert. Man erhält 21,5 g (52% der Theorie) 4-Methoxycarbonyl-2-methyl-thiophen-3-sulfonamid.

Analog zu Beispiel (II-1) können beispielsweise auch die folgenden Verbindungen der allgemeinen Formel (II) hergestellt werden:
4-Ethoxycarbonyl-2-methyl-thiophen-3-sulfonamid,
4-n-Propoxycarbonyl-2-methyl-thiophen-3-sulfonamid,
4-i-Propoxycarbonyl-2-methyl-thiophen-3-sulfonamid,
4-Methoxycarbonyl-2-ethyl-thiophen-3-sulfonamid,
4-Ethoxycarbonyl-2-ethyl-thlophen-3-sulfonamid,
4-n-Propoxycarbonyl-2-ethyl-thiophen-3-sulfonamid,
4-i-Propoxycarbonyl-2-ethyl-thiophen-3-sulfonamid,
4-Methoxycarbonyl-2-n-propyl-thiophen-3-sulfonamid,
4-Ethoxycarbonyl-2-n-propyl-thiophen-3-sulfonamid,
4-n-Propoxycarbonyl-2-n-propyl-thiophen-3-sulfonamid,
4-i-Propoxycarbonyl-2-n-propyl-thiophen-3-sulfonamid,
4-Methoxycarbonyl-2-i-propyl-thiophen-3-sulfonamid,
4-Ethoxycarbonyl-2-i-propyl-thiophen-3-sulfonamid,
4-n-Propoxycarbonyl-2-i-propyl-thiophen-3-sulfonamid,
4-i-Propoxycarbonyl-2-i-propyl-thiophen-3-sulfonamid.

### Ausgangsstoffe der Formel (VI):

### Beispiel (VI-I)

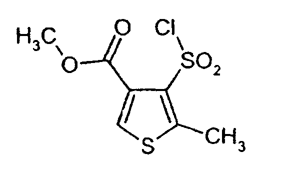

Eine Lösung von 19,9 g (0,29 Mol) Natriumnitrit in 60 ml Wasser wird bei 0°C bis 5°C tropfenweise unter Rühren zu einer Lösung von 42,7 g (0,25 Mol) 3-Amino-2-methyl-thiophen-4-carbonsäure-methylester in 75 ml 10%iger wässriger Salzsäure gegeben. Die Reaktionsmischung wird 60 Minuten bei 0°C bis 5°C gerührt. Anschließend wird der Nitrit-Überschuß mit Amidosulfonsäure beseitigt. Die Mischung wird dann bei 0°C bis 5°C tropfenweise unter Rühren zu einer Lösung von 35 g (0,55 Mol) Schwefeldioxid in 300 ml Methylenchlorid gegeben. Nach Zugabe von 1,5 g Kupfer(I)-chlorid und 1,5 g Dodecyl-trimethylammonium-bromid wird die Reaktionsmischung 60 Minuten bei 40°C und weiter 12 Stunden bei 20°C gerührt. Anschließend werden 18 ml 35%ige wässrige Salzsäure dazu gegeben, die Mischung 4 Stunden bei 20°C gerührt und dann die Phasen getrennt. Die wässrige Phase wird mit Methylenchlorid nachextrahiert, die vereinigten organischen Phasen mit Wasser gewaschen, mit Magnesiumsulfat getrocknet und filtriert. Das Filtrat wird im Wasserstrahlvakuum eingeengt und der Rückstand aus Hexan kristallisiert.

Man erhält 51,7 g (81% der Theorie) 4-Methoxycarbonyl-2-methyl-thiophen-3-sulfonsäurechlorid.

Analog zu Beispiel (VI-I) können beispielsweise auch die folgenden Verbindungen der allgemeinen Formel (VI) hergestellt werden:
4-Ethoxycarbonyl-2-methyl-thiophen-3-sulfonsäurechlorid,
4-n-Propoxycarbonyl-2-methyl-thiophen-3-sulfonsäurechlorid,
4-i-Propoxycarbonyl-2-methyl-thiophen-3-sulfonsäurechlorid,
4-Methoxycarbonyl-2-ethyl-thiophen-3-sulfonsäurechlorid,
4-Ethoxycarbonyl-2-ethyl-thiophen-3-sulfonsäurechlorid,
4-n-Propoxycarbonyl-2-ethyl-thiophen-3-sulfonsäurechlorid,
4-i-Propoxycarbonyl-2-ethyl-thiophen-3-sulfonsäurechlorid,
4-Methoxycarbonyl-2-n-propyl-thiophen-3-sulfonsäurechlorid,
4-Ethoxycarbonyl-2-n-propyl-thiophen-3-sulfonsäurechlorid,
4-n-Propoxycarbonyl-2-n-propyl-thiophen-3-sulfonsäurechlorid,
4-i-Propoxycarbonyl-2-n-propyl-thiophen-3-sulfonsäurechlorid,
4-Methoxycarbonyl-2-i-propyl-thiophen-3-sulfonsäurechlorid,
4-Ethoxycarbonyl-2-i-propyl-thiophen-3-sulfonsäurechlorid,
4-n-Propoxycarbonyl-2-i-propyl-thiophen-3-sulfonsäurechlorid,
4-i-Propoxycarbonyl-2-i-propyl-thiophen-3-sulfonsäurechlorid.

### Ausgansstoffe der Formel (X):

### Beispiel (X-1)

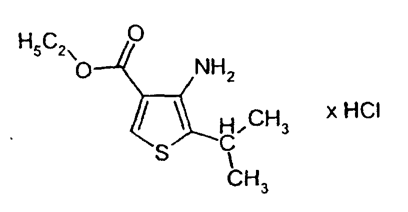

### Stufe 1

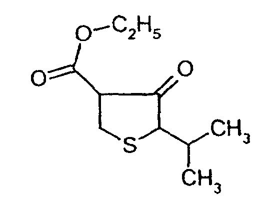

61 g einer 20 %igen Lösung von Natriumethylat in Ethanol (213 mMol NaOCH₃) werden im Wasserstrahlvakuum zur Trockne eingedampft. Der Rückstand wird in 80 ml Toluol aufgenommen und dann werden 28,6 g (109 mMol) 2-(2-Ethoxycarbonyl-ethylthio)-3-methyl-butansäure-ethylester dazu gegeben und die Reaktionsmischung wird 12 Stunden bei 70°C bis 80°C gerührt. Nach Abkühlen auf Raumtemperatur wird die Mischung auf Eiswasser gegossen und dann mit konz. Salzsäure angesäuert. Anschließend wird die organische Phase abgetrennt, die wässrige Phase mit Diethylether nachextrahiert, die organischen Phasen vereinigt, mit Magnesiumsulfat getrocknet und filtriert. Das Filtrat wird im Wasserstrahlvakuum eingeengt und der Rückstand durch Destillation unter vermindertem Druck gereinigt.

Man erhält 22,6 g (96% der Theorie) 5-i-Propyl-4-oxo-tetrahydrothiophen-3-carbonsäure-ethylester vom Siedepunkt 115°C (bei 0,5 mbar).

### Stufe 2

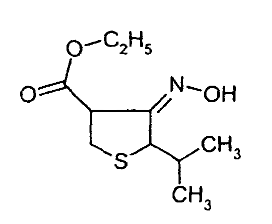

Eine Mischung aus 38 g (176 mMol) 5-i-Propyl-4-oxo-tetrahydrothiophen-3-carbonsäure-ethylester, 35 g Hydroxylamin-Hydrochlorid, 53 g Bariumcarbonat und 300 ml Ethanol wird 12 Stunden unter Rückfluß erhitzt und anschließend heiß filtriert. Das Filtrat wird im Wasserstrahlvakuum eingeengt, der Rückstand in Diethylether aufgenommen, mit Wasser gewaschen, mit Magnesiumsulfat getrocknet und filtriert. Vom Filtrat wird das Lösungsmittel unter vermindertem Druck sorgfältig abdestilliert.

Man erhält 34,2 g (86 % der Theorie) 4-Hydroximino-5-i-propyl-dihydro-5Hthiophen-3-carbonsäure-ethylester als Öl, welches ohne weitere Reinigung weiter umgesetzt werden kann.

### Stufe 3

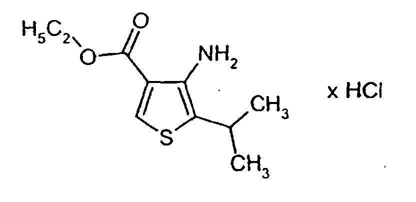

33 g (143 mMol) 4-Hydroximino-5-i-prapyi-dihydro-5H-thiophen-3-carbonsäureethylester werden in 250 ml Diethylether gelöst und unter Eiskühlung wird Hydrogenchlorid 20 Minuten lang (bis zur Sättigung) eingeleitet. Man läßt die Mischung zwei Tage lang bei Raumtemperatur (ca. 20°C) stehen, engt dann im Wasserstrahlvakuum ein und kristallisiert den Rückstand aus Aceton.

Man erhält 13 g (37 % der Theorie) 4-Amino-5-i-propyl-thiophen-3-carbonsäureethylester-Hydrochlorid als festes Produkt.

### Beispiel (X-2)

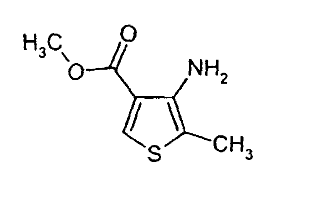

### Stufe 1

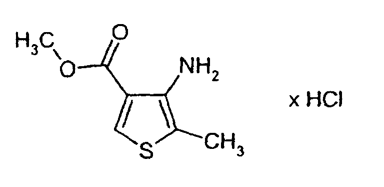

Eine Mischung aus 310 g (1,78 Mol) 5-Methyl-4-oxo-tetrahydrothiophen-3-carbonsäure-methylester, 155 g (2,27 Mol) Hydroxylamin-Hydrochlorid und 900 ml Acetonitril wird 60 Minuten unter Rückfluß erhitzt. Nach Abkühlen auf Raumtemperatur (ca. 20°C) wird das kristallin angefallene Produkt durch Absaugen isoliert.

Man erhält 335 g (91% der Theorie) 4-Amino-5-methyl-thiophen-3-carbonsäuremethylester-Hydrochlorid vom Schmelzpunkt 132°C.

### Stufe 2

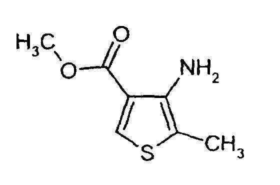

273 g (1,62 Mol) 4-Amino-5-methyl-thiophen-3-carbonsäure-methylester-Hydrochlorid werden in 1 Liter Wasser gelöst und mit 2 Liter Methylenchlorid unterschichtet. Unter starkem Rühren werden dann 125 g Natriumhydrogencarbonat dazu gegeben und die Mischung wird noch weitere 15 Minuten gerührt. Die organische Phase wird abgetrennt, mit Magnesiumsulfat getrocknet und filtriert. Das Filtrat wird im Wasserstrahlvakuum eingeengt, der Rückstand mit Petrolether digeriert und das kristalline Produkt durch Absaugen isoliert.

Man erhält 148 g (53 % der Theorie) 4-Amino-5-methyl-thiophen-3-carbonsäuremethylester vom Schmelzpunkt 78°C.

Analog zu den Beispielen (X-1) und (X-2) können beispielsweise auch die folgenden Verbindungen der allgemeinen Formel (X) hergestellt werden:
4-Amino-5-methyl-thiophen-3-carbonsäure-ethylester
   (Fp.: 50°C, Hydrochlorid: Fp.: 143°C),
4-Amino-5-methyl-thiophen-3-carbonsäure-n-propylester,
4-Amino-5-methyl-thiophen-3-carbonsäure-i-propylester,
4-Amino-5-ethyl-thiophen-3-carbonsäure-methylester,
4-Amino-5-ethyl-thiophen-3-carbonsäure-ethylester,
4-Amino-5-ethyl-thiophen-3-carbonsäure-n-propylester
   (Öl, Hydrochlorid: Fp.: 140°C),
4-Amino-5-ethyl-thiophen-3-carbonsäure-i-propylester
   (Öl, Hydrochlorid: Fp.: 142°C),
4-Amino-5-n-propyl-thiophen-3-carbonsäure-methylester,
4-Amino-5-n-propyl-thiophen-3-carbonsäure-ethylester,
4-Amino-5-n-propyl-thiophen-3-carbonsäure-n-propylester,
4-Amino-5-n-propyl-thiophen-3-carbonsäure-i-propylester,
4-Amino-5-i-propyl-thiophen-3-carbonsäure-methylester,
4-Amino-5-i-propyl-thiophen-3-carbonsäure-n-propylester,
4-Amino-5-i-propyl-thiophen-3-carbonsäure-i-propylester,
sowie jeweils die entsprechenden Hydrochloride.

### Anwendungsbeispiele:

### Beispiel A

### Pre-emergence-Test

| | |
|---|---|
| Lösungsmittel | 5 Gewichtsteile Aceton |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät. Nach ca. 24 Stunden wird der Boden so mit der Wirkstoffzubereitung besprüht, daß die jeweils gewünschte Wirkstoffmenge pro Flächeneinheit ausgebracht wird. Die Konzentration der Spritzbrühe wird so gewählt, daß in 1000 Liter Wasser pro Hektar die jeweils gewünschte Wirkstoffmenge ausgebracht wird.

Nach drei Wochen wird der Schädigungsgrad der Pflanzen bonitiert in % Schädigung im Vergleich zur Entwicklung der unbehandelten Kontrolle.

Es bedeuten:

| | |
|---|---|
| 0 % = | keine Wirkung (wie unbehandelte Kontrolle) |
| 100 % = | totale Vernichtung |

In diesem Test zeigen beispielsweise die Verbindungen gemäß Herstellungsbeispiel 1, 2, 3, 4, 5, 6, 7, 8, 9, 10, 11 und 12 bei teilweise guter Verträglichkeit gegenüber Kulturpflanzen, wie z.B. Baumwolle, Mais und Weizen, sehr starke Wirkung gegen Unkräuter.

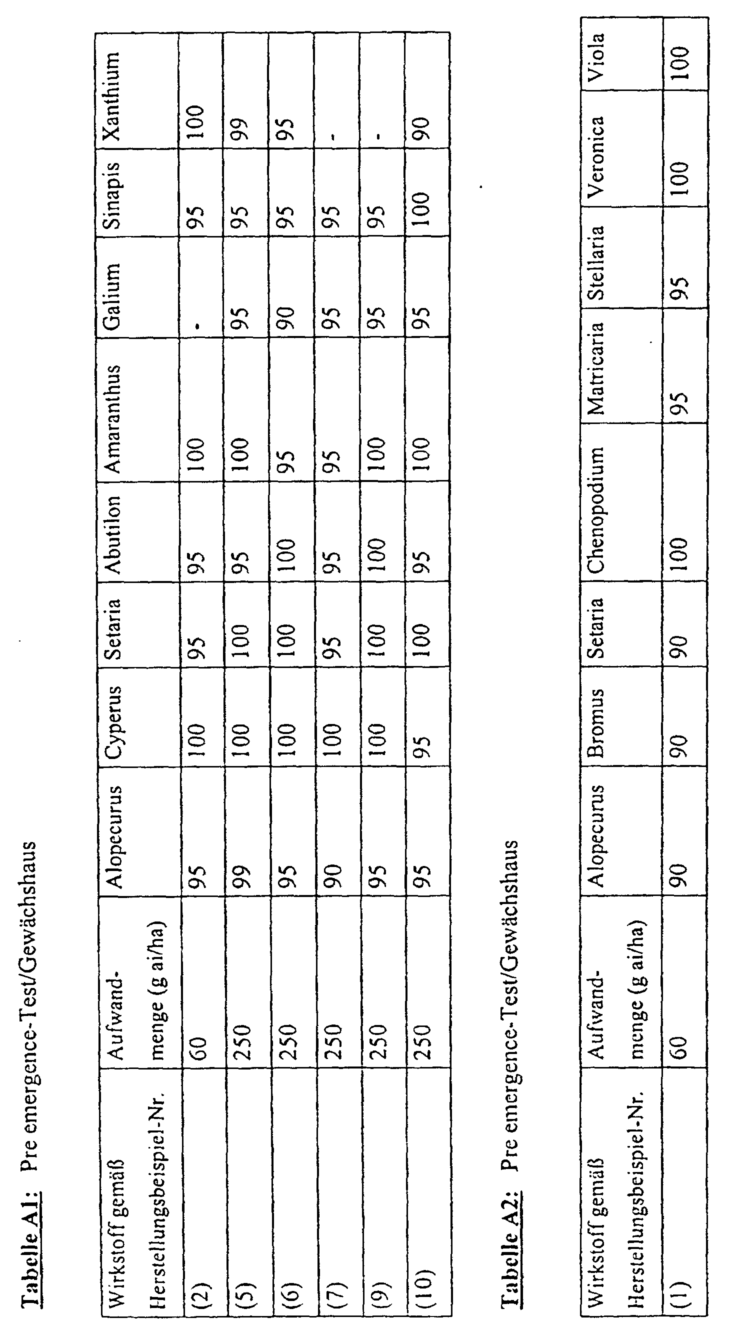

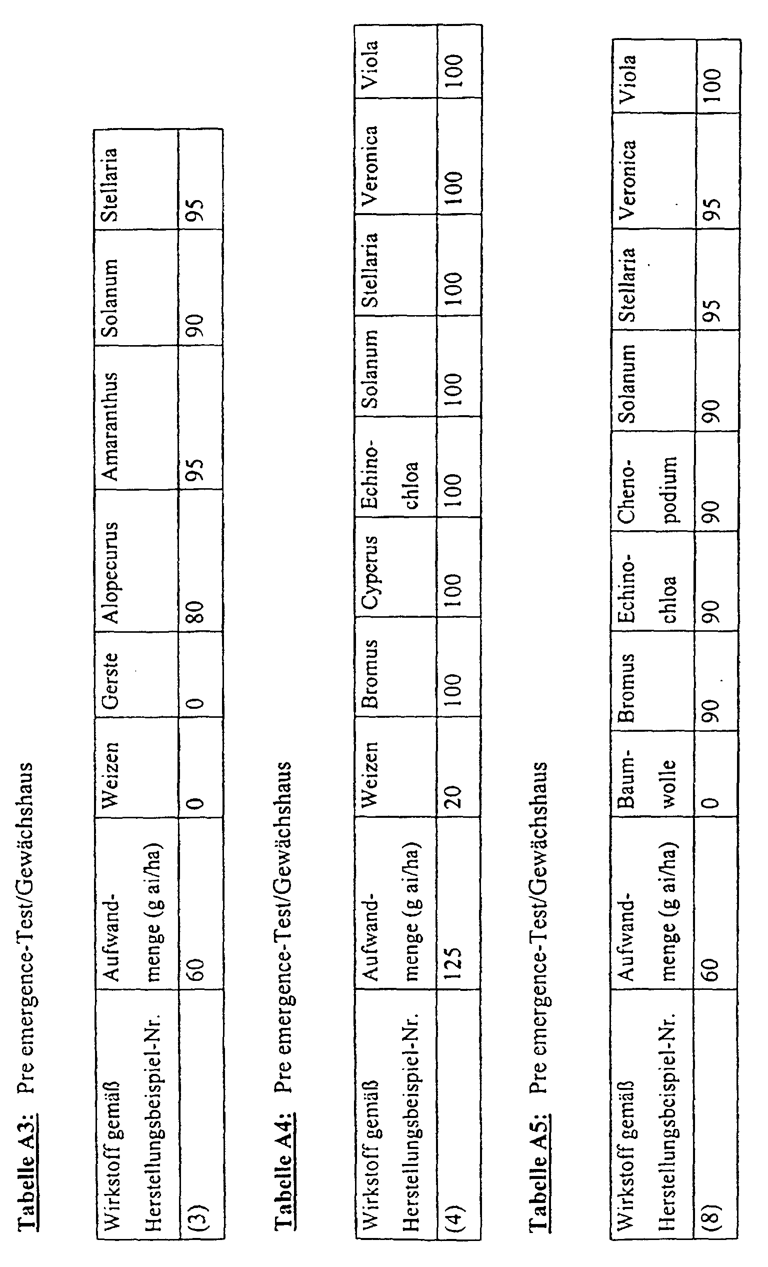

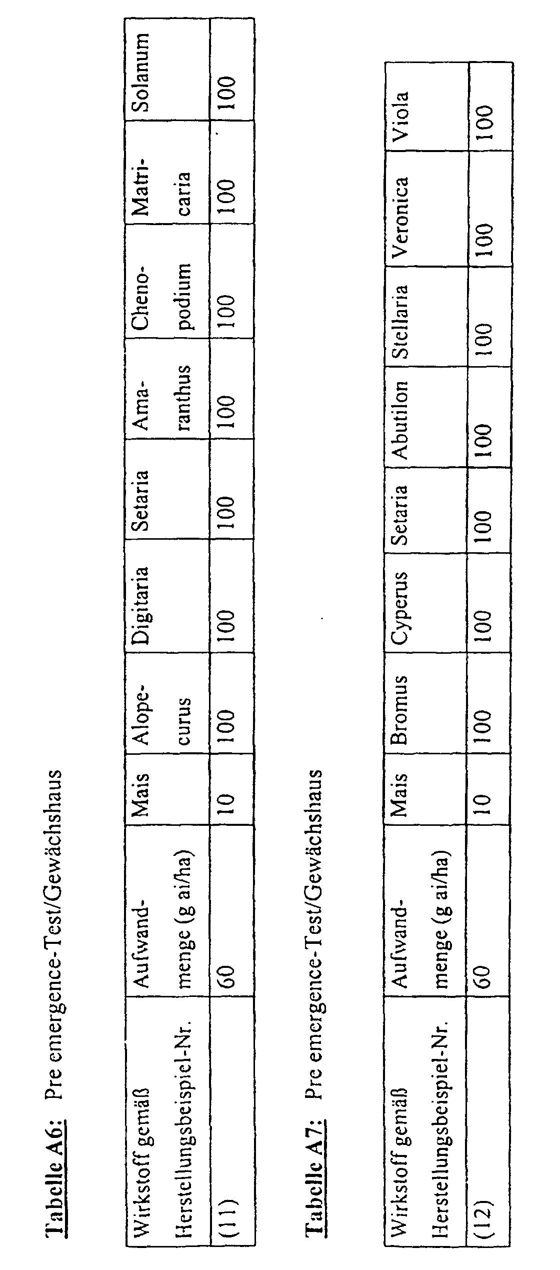

### Beispiel B

### Post-emergence-Test

| | |
|---|---|
| Lösungsmittel | 5 Gewichtsteile Aceton |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 1000 1 Wasser/ha die jeweils gewünschten Wirkstoffmengen ausgebracht werden.

Nach drei Wochen wird der Schädigungsgrad der Pflanzen bonitiert in % Schädigung im Vergleich zur Entwicklung der unbehandelten Kontrolle.

Es bedeuten:

| | |
|---|---|
| 0 % = | keine Wirkung (wie unbehandelte Kontrolle) |
| 100 % = | totale Vernichtung |

In diesem Test zeigen beispielsweise die Verbindungen gemäß Herstellungsbeispiel 1, 2, 3, 4, 5, 6, 7, 8, 9, 10, 11 und 12 bei teilweise guter Verträglichkeit gegenüber Kulturpflanzen, wie z.B. Gerste und Weizen, sehr starke Wirkung gegen Unkräuter.

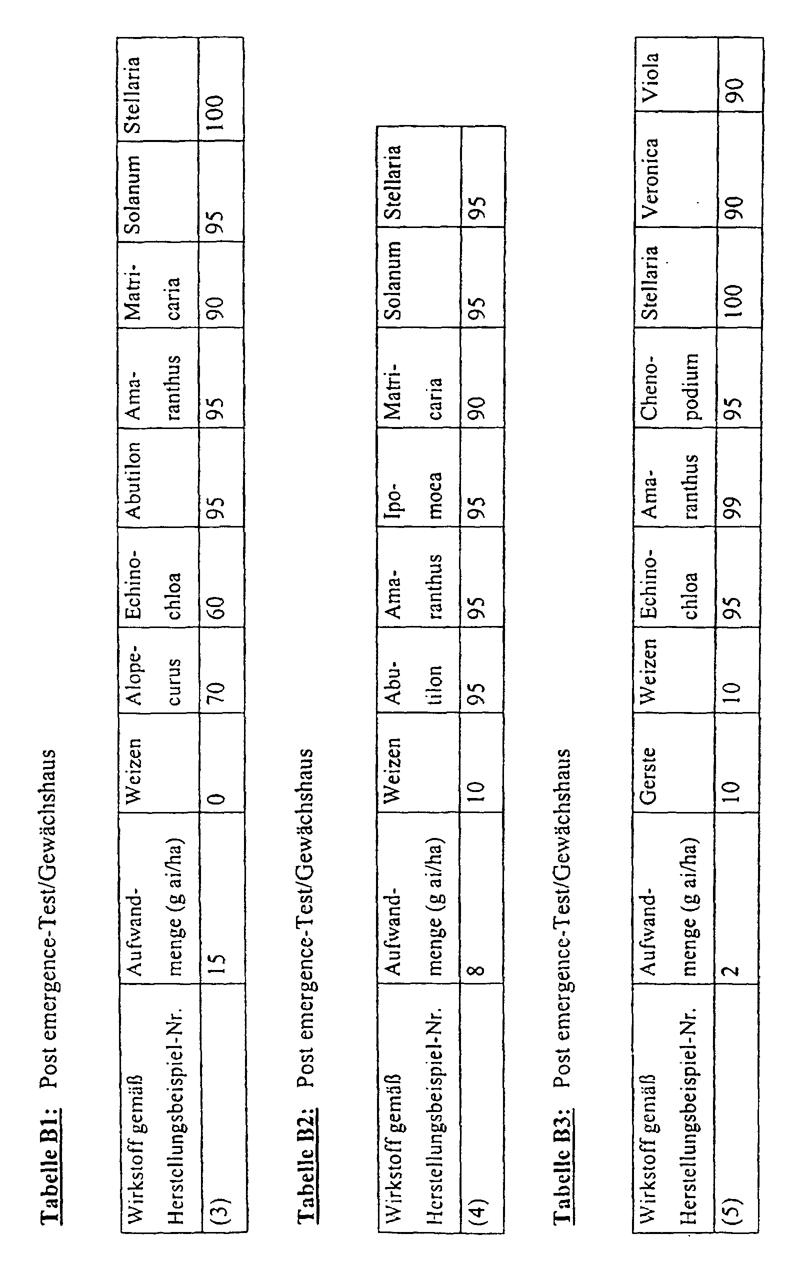

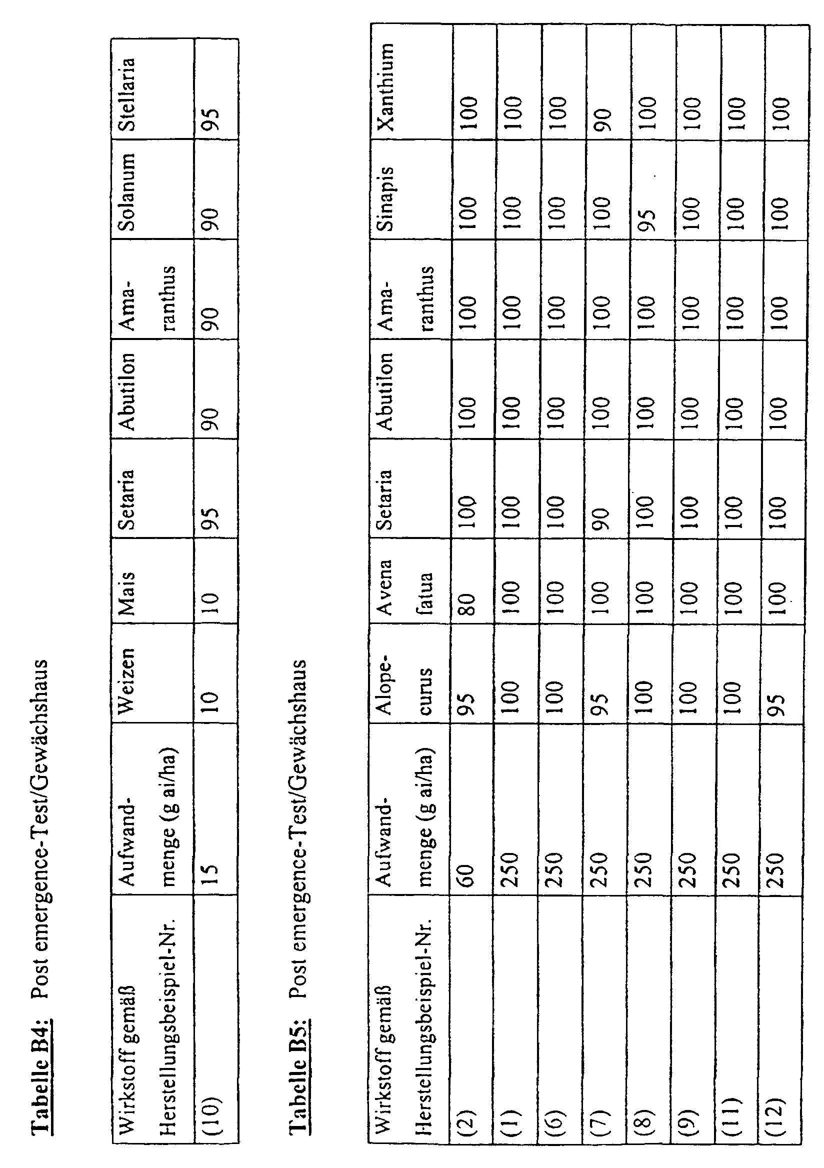

## Patentansprüche

1. Verbindungen der allgemeinen Formel (I) 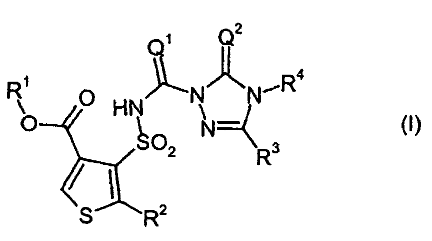 in welcher
Q¹ für O (Sauerstoff) oder S (Schwefel) steht,
Q² für O (Sauerstoff) oder S (Schwefel) steht,
R¹ für gegebenenfalls durch Cyano, Halogen oder C₁-C₄-Alkoxy substituiertes Alkyl mit 1 bis 6 Kohlenstoffatomen, für jeweils gegebenenfalls durch Cyano oder Halogen substituiertes Alkenyl oder Alkinyl mit jeweils 2 bis 6 Kohlenstoffatomen, für jeweils gegebenenfalls durch Cyano, Halogen oder C₁-C₄-Alkyl substituiertes Cycloalkyl oder Cycloalkylalkyl mit jeweils 3 bis 6 Kohlenstoffatomen in der Cycloalkylgruppe und gegebenenfalls 1 bis 4 Kohlenstoffatomen im Alkylteil, für jeweils gegebenenfalls durch Nitro, Cyano, Halogen, C₁-C₄-Alkyl oder C₁-C₄-Alkoxy substituiertes Aryl oder Arylalkyl mit jeweils 6 oder 10 Kohlenstoffatomen in der Arylgruppe und gegebenenfalls 1 bis 4 Kohlenstoffatomen im Alkylteil, oder für jeweils gegebenenfalls durch Nitro, Cyano, Halogen, C₁-C₄-Alkyl oder C₁-C₄-Alkoxy substituiertes Heterocyclyl oder Heterocyclylalkyl mit jeweils bis zu 6 Kohlenstoffatomen und zusätzlich 1 bis 4 Stickstoffatomen und/oder 1 bis 2 Sauerstoff- oder Schwefelatomen in der Heterocyclylgruppe und gegebenenfalls 1 bis 4 Kohlenstoffatomen im Alkylteil steht,
R² für Wasserstoff, Cyano, Nitro, Halogen, für jeweils gegebenenfalls durch Cyano, Halogen oder C₁-C₄-Alkoxy substituiertes Alkyl, Alkoxy, Alkylthio, Alkylsulfinyl oder Alkylsulfonyl mit jeweils 1 bis 6 Kohlenstoffatomen in der Alkylgruppe, oder für jeweils gegebenenfalls durch Cyano oder Halogen substituiertes Alkenyl, Alkinyl, Alkenyloxy oder Alkinyloxy mit jeweils 2 bis 6 Kohlenstoffatomen in der Alkenyl- oder Alkinylgruppe steht,
R³ für Wasserstoff, Hydroxy, Mercapto, Amino, Cyano, Fluor, Chlor, Brom, Iod, für gegebenenfalls durch Fluor, Chlor, Brom, Cyano, C₁-C₄-Alkoxy, C₁-C₄-Alkyl-carbonyl oder C₁-C₄-Alkoxy-carbonyl substituiertes Alkyl mit 1 bis 6 Kohlenstoffatomen, für jeweils gegebenenfalls durch Fluor, Chlor und/oder Brom substituiertes Alkenyl oder Alkinyl mit jeweils 2 bis 6 Kohlenstoffatomen, für jeweils gegebenenfalls durch Fluor, Chlor, Cyano, C₁-C₄-Alkoxy oder C₁-C₄-Alkoxycarbonyl substituiertes Alkoxy, Alkylthio, Alkylamino oder Alkylcarbonylamino mit jeweils 1 bis 6 Kohlenstoffatomen in der Alkylgruppe, für Alkenyloxy, Alkinyloxy, Alkenylthio, Alkinylthio, Alkenylamino oder Alkinylamino mit jeweils 3 bis 6 Kohlenstoffatomen in der Alkenyl- oder Alkinylgruppe, für Dialkylamino mit jeweils 1 bis 4 Kohlenstoffatomen in den Alkylgruppen, für jeweils gegebenenfalls durch Methyl und/oder Ethyl substituiertes Aziridino, Pyrrolidino, Piperidino oder Morpholino, für jeweils gegebenenfalls durch Fluor, Chlor, Brom, Cyano und/oder C₁-C₄-Alkyl substituiertes Cycloalkyl, Cycloalkenyl, Cycloalkyloxy, Cycloalkylthio, Cycloalkylamino, Cycloalkylalkyl, Cycloalkylalkoxy, Cycloalkylalkylthio oder Cycloalkylalkylamino mit jeweils 3 bis 6 Kohlenstoffatomen in der Cycloalkyl- bzw. Cycloalkenylgruppe und gegebenenfalls 1 bis 4 Kohlenstoffatomen im Alkylteil, oder für jeweils gegebenenfalls durch Fluor, Chlor, Brom, Cyano, Nitro, C₁-C₄-Alkyl, Trifluormethyl, C₁-C₄-Alkoxy und/oder C₁-C₄-Alkoxy-carbonyl substituiertes Aryl, Arylalkyl, Aryloxy, Arylalkoxy, Arylthio, Arylalkylthio, Arylamino oder Arylalkylamino mit jeweils 6 oder 10 Kohlenstoffatomen in der Arylgruppe und gegebenenfalls 1 bis 4 Kohlenstoffatomen im Alkylteil steht, und
R⁴ für Wasserstoff, Hydroxy, Amino, Cyano, für C₂-C₁₀-Alkylidenamino, für gegebenenfalls durch Fluor, Chlor, Brom, Cyano, C₁-C₄-Alkoxy, C₁-C₄-Alkyl-carbonyl oder C₁-C₄-Alkoxy-carbonyl substituiertes Alkyl mit 1 bis 6 Kohlenstoffatomen, für jeweils gegebenenfalls durch Fluor, Chlor und/oder Brom substituiertes Alkenyl oder Alkinyl mit jeweils 2 bis 6 Kohlenstoffatomen, für jeweils gegebenenfalls durch Fluor, Chlor, Brom, Cyano, C₁-C₄-Alkoxy oder C₁-C₄-Alkoxy-carbonyl substituiertes Alkoxy, Alkylamino oder Alkylcarbonylamino mit jeweils 1 bis 6 Kohlenstoffatomen in der Alkylgruppe, für Alkenyloxy mit 3 bis 6 Kohlenstoffatomen, für Dialkylamino mit jeweils 1 bis 4 Kohlenstoffatomen in den Alkylgruppen, für jeweils gegebenenfalls durch Fluor, Chlor, Brom, Cyano und/oder C₁-C₄-Alkyl substituiertes Cycloalkyl, Cycloalkylamino oder Cycloalkylalkyl mit jeweils 3 bis 6 Kohlenstoffatomen in der Alkylgruppe und gegebenenfalls 1 bis 4 Kohlenstoffatomen im Alkylteil, oder für jeweils gegebenenfalls durch Fluor, Chlor, Brom, Cyano, Nitro, C₁-C₄-Alkyl, Trifluormethyl und/oder C₁-C₄-Alkoxy substituiertes Aryl oder Arylalkyl mit jeweils 6 oder 10 Kohlenstoffatomen in der Arylgruppe und gegebenenfalls 1 bis 4 Kohlenstoffatomen im Alkylteil steht, oder
R³ und R⁴ zusammen für gegebenenfalls verzweigtes Alkandiyl mit 3 bis 6 Kohlenstoffatomen stehen,
sowie die Salze dieser Verbindungen.

2. Verbindungen gemäß Anspruch 1, **dadurch gekennzeichnet, daß**
Q¹ für O (Sauerstoff) oder S (Schwefel) steht,
Q² für O (Sauerstoff) oder S (Schwefel) steht,
R¹ für jeweils gegebenenfalls durch Cyano, Fluor, Chlor, Methoxy oder Ethoxy substituiertes Methyl, Ethyl, n- oder i-Propyl, n-, i-, s- oder t-Butyl, für jeweils gegebenenfalls durch Cyano, Fluor oder Chlor substituiertes Propenyl, Butenyl, Propinyl oder Butinyl, für jeweils gegebenenfalls durch Cyano, Fluor, Chlor, Methyl oder Ethyl substituiertes Cyclopropyl, Cyclobutyl, Cyclopentyl, Cyclohexyl, Cyclopropylmethyl, Cyclobutylmethyl, Cyclopentylmethyl oder Cyclohexylmethyl, für jeweils gegebenenfalls durch Cyano, Fluor, Chlor, Brom, Methyl, Ethyl, n- oder i-Propyl, Trifluormethyl, Methoxy, Ethoxy, n- oder i-Propoxy, Difluormethoxy oder Trifluormethoxy substituiertes Phenyl, Phenylmethyl oder Phenylethyl, oder für jeweils gegebenenfalls durch Cyano, Fluor, Chlor, Brom, Methyl, Ethyl, n- oder i-Propyl, Methoxy, Ethoxy, n- oder i-Propoxy substituiertes Heterocyclyl oder Heterocyclylmethyl steht, wobei die Heterocyclylgruppe jeweils aus der Reihe Oxetanyl, Thietanyl, Furyl, Tetrahydrofuryl, Thienyl, Tetrahydrothienyl ausgewählt ist,
R² für Wasserstoff, Cyano, Fluor, Chlor, Brom, für jeweils gegebenenfalls durch Cyano, Fluor, Chlor, Methoxy oder Ethoxy substituiertes Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, oder für jeweils gegebenenfalls durch Cyano, Fluor oder Chlor substituiertes Propenyl, Butenyl, Propinyl, Butinyl, Propenyloxy, Butenyloxy, Propinyloxy oder Butinyloxy steht,
R³ für Wasserstoff, Hydroxy, Mercapto, Amino, Cyano, Fluor, Chlor, Brom, für jeweils gegebenenfalls durch Fluor, Chlor, Cyano, Methoxy, Ethoxy, n- oder i-Propoxy, Acetyl, Propionyl, n- oder i-Butyroyl, Methoxycarbonyl, Ethoxycarbonyl, n- oder i-Propoxycarbonyl substituiertes Methyl, Ethyl, n- oder i-Propyl, n-, i-, s- oder t-Butyl, für jeweils gegebenenfalls durch Fluor, Chlor und/oder Brom substituiertes Ethenyl, Propenyl, Butenyl, Ethinyl, Propinyl oder Butinyl, für jeweils gegebenenfalls durch Fluor, Chlor, Cyano, Methoxy, Ethoxy, n- oder i-Propoxy, Methoxycarbonyl, Ethoxycarbonyl, n- oder i-Propoxy-carbonyl substituiertes Methoxy, Ethoxy, n- oder i-Propoxy, n-, i-, s- oder t-Butoxy, Methylthio, Ethylthio, n- oder i-Propylthio, n-, i-, s- oder t-Butylthio, Methylamino, Ethylamino, n- oder i-Propylamino, n-, i-, s- oder t-Butylamino, Acetylamino oder Propionylamino, für Propenyloxy, Butenyloxy, Ethinyloxy, Propinyloxy, Butinyloxy, Propenylthio, Butenylthio, Propinylthio, Butinylthio, Propenylamino, Butenylamino, Propinylamino oder Butinylamino, für Dimethylamino, Diethylamino oder Dipropylamino, für jeweils gegebenenfalls durch Fluor, Chlor, Methyl und/oder Ethyl substituiertes Cyclopropyl, Cyclobutyl, Cyclopentyl, Cyclohexyl, Cyclopentenyl, Cyclohexenyl, Cyclopropyloxy, Cyclobutyloxy, Cyclopentyloxy, Cyclohexyloxy, Cyclopropylthio, Cyclobutylthio, Cyclopentylthio, Cyclohexylthio, Cyclopropylamino, Cyclobutylamino, Cyclopentylamino, Cyclohexylamino, Cyclopropylmethyl, Cyclobutylmethyl, Cyclopentylmethyl, Cyclohexylmethyl, Cyclopropylmethoxy, Cyclobutylmethoxy, Cyclopentylmethoxy, Cyclohexylmethoxy, Cyclopropylmethylthio, Cyclobutylmethylthio, Cyclopentylmethylthio, Cyclohexylmethylthio, Cyclopropylmethylamino, Cyclobutylmethylamino, Cyclopentylmethylamino oder Cyclohexylmethylamino, oder für jeweils gegebenenfalls durch Fluor, Chlor, Brom, Methyl, Trifluormethyl, Methoxy oder Methoxy-carbonyl substituiertes Phenyl, Benzyl, Phenoxy, Benzyloxy, Phenylthio, Benzylthio, Phenylamino oder Benzylamino steht, und
R⁴ für Wasserstoff, Hydroxy, Amino, für jeweils gegebenenfalls durch Fluor, Chlor, Cyano, Methoxy oder Ethoxy substituiertes Methyl, Ethyl, n- oder i-Propyl, n-, i-, s- oder t-Butyl, für jeweils gegebenenfalls durch Fluor, Chlor und/oder Brom substituiertes Ethenyl, Propenyl, Butenyl, Propinyl oder Butinyl, für jeweils gegebenenfalls durch Fluor, Chlor, Cyano, Methoxy oder Ethoxy substituiertes Methoxy, Ethoxy, n- oder i-Propoxy, n-, i-, s- oder t-Butoxy, Methylamino, Ethylamino, n- oder i-Propylamino, n-, i-, s- oder t-Butylamino, für Propenyloxy oder Butenyloxy, für Dimethylamino oder Diethylamino, für jeweils gegebenenfalls durch Fluor, Chlor, Methyl und/oder Ethyl substituiertes Cyclopropyl, Cyclobutyl, Cyclopentyl, Cyclohexyl, Cyclopropylamino, Cyclobutylamino, Cyclopentylamino, Cyclohexylamino, Cyclopropylmethyl, Cyclobutylmethyl, Cyclopentylmethyl oder Cyclohexylmethyl, oder für jeweils gegebenenfalls durch Fluor, Chlor, Methyl, Trifluormethyl und/oder Methoxy substituiertes Phenyl oder Benzyl steht, oder
R³ und R⁴ zusammen für Trimethylen (Propan-1,3-diyl), Tetramethylen (Butan-1,4-diyl) oder Pentamethylen (Pentan-1,5-diyl) stehen,
sowie die Natrium-, Kalium-, Magnesium-, Calcium-, Ammonium-, C₁-C₄-Alkyl-ammonium-, Di-(C₁-C₄-alkyl)-ammonium-, Tri-(C₁-C₄-alkyl)-ammonium-, Tetra-(C₁-C₄-alkyl)-ammonium, Tri-(C₁-C₄-alkyl)-sulfonium-, C₅- oder C₆-Cycloalkyl-ammonium- und Di-(C₁-C₂-alkyl)-benzyl-ammonium-Salze dieser Verbindungen.

3. Verbindungen gemäß Anspruch 1, **dadurch gekennzeichnet, daß**
Q¹ für O (Sauerstoff) steht,
Q² für O (Sauerstoff) steht,
R¹ für jeweils gegebenenfalls durch Fluor, Chlor, Methoxy oder Ethoxy substituiertes Methyl, Ethyl, n- oder 1-Propyl steht,
R² für Fluor, Chlor, Brom oder für jeweils gegebenenfalls durch Fluor, Chlor, Methoxy oder Ethoxy substituiertes Methyl, Ethyl, n- oder i-Propyl steht,
R³ für Wasserstoff, Chlor, Brom, für jeweils gegebenenfalls durch Fluor, Chlor, Methoxy, Ethoxy, n- oder i-Propoxy substituiertes Methyl, Ethyl, n- oder i-Propyl, für jeweils gegebenenfalls durch Fluor oder Chlor substituiertes Ethenyl, Propenyl, Butenyl, Propinyl oder Butinyl, für jeweils gegebenenfalls durch Fluor, Chlor, Methoxy, Ethoxy, n- oder i-Propoxy substituiertes Methoxy, Ethoxy, n- oder i-Propoxy, Methylthio, Ethylthio, n- oder i-Propylthio, Methylamino, Ethylamino, n- oder i-Propylamino, für Propenyloxy, Propinyloxy, Propenylthio, Propinylthio, Propenylamino oder Propinylamino, für Dimethylamino oder Diethylamino, für jeweils gegebenenfalls durch Fluor, Chlor oder Methyl substituiertes Cyclopropyl, Cyclopropyloxy, Cyclopropylmethyl oder Cyclopropylmethoxy steht, und
R⁴ für jeweils gegebenenfalls durch Fluor, Chlor, Methoxy oder Ethoxy substituiertes Methyl, Ethyl, n- oder i-Propyl, für jeweils gegebenenfalls durch Fluor oder Chlor substituiertes Ethenyl, Propenyl oder Propinyl, für jeweils gegebenenfalls durch Fluor, Chlor, Methoxy oder Ethoxy substituiertes Methoxy, Ethoxy, n- oder i-Propoxy, für Methylamino, oder für Cyclopropyl steht,
sowie die Natrium-, Kalium-, Magnesium-, Calcium-, Ammonium-, C₁-C₄-Alkyl-ammonium-, Di-(C₁-C₄-alkyl)-ammonium-, Tri-(C₁-C₄-alkyl)-ammonium-, Tetra-(C₁-C₄-alkyl)-ammonium, Tri-(C₁-C₄-alkyl)-sulfonium-, C₅- oder C₆-Cycloalkyl-ammonium- und Di-(C₁-C₂-alkyl)-benzyl-ammonium-Salze dieser Verbindungen.

4. Verfahren zum Herstellen von Verbindungen gemäß einem der Ansprüche 1 bis 3, **dadurch gekennzeichnet, daß**
(a) substituierte Thiophen-3-sulfonamide der allgemeinen Formel (II) 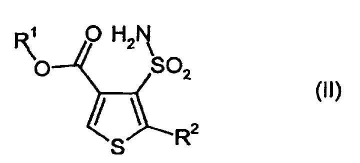 in welcher
R¹ und R² die in einem der Ansprüche 1 bis 3 angegebene Bedeutung haben,
mit substituierten Triazolin(thi)onen der allgemeinen Formel (III) 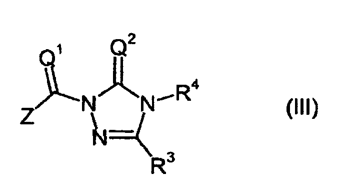 in welcher
Q¹, Q², R³ und R⁴ die in einem der Ansprüche 1 bis 3 angegebene Bedeutung haben und
Z für Halogen, Alkoxy, Aryloxy oder Arylalkoxy steht,
gegebenenfalls in Gegenwart eines Reaktionshilfsmittels und gegebenenfalls in Gegenwart eines Verdünnungsmittels umgesetzt werden,
oder daß
(b) substituierte Thien-3-yl-sulfonyl-iso(thio)cyanate der allgemeinen Formel (IV) 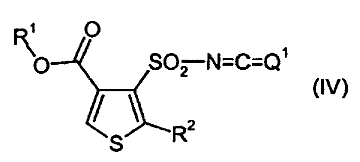 in welcher
Q¹, R¹ und R² die in einem der Ansprüche 1 bis 3 angegebene Bedeutung haben,
mit Triazolin(thi)onen der allgemeinen Formel (V) 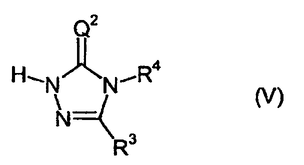 in welcher
Q², R⁴ und R⁵ die in einem der Ansprüche 1 bis 3 angegebene Bedeutung haben,
gegebenenfalls in Gegenwart eines Reaktionshilfsmittels und gegebenenfalls in Gegenwart eines Verdünnungsmittels umgesetzt werden,
oder daß
(c) substituierte Thiophen-3-sulfonsäurechloride der allgemeinen Formel (VI) 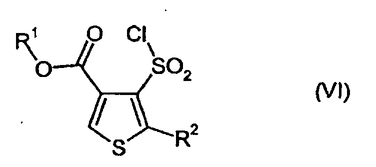 in welcher
R¹ und R² die in einem der Ansprüche 1 bis 3 angegebene Bedeutung haben,
mit Triazolin(thi)onen der allgemeinen Formel (V) 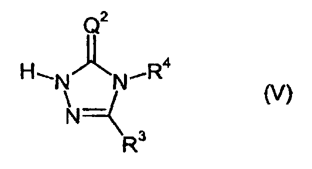 in welcher
Q², R⁴ und R⁵ die in einem der Ansprüche 1 bis 3 angegebene Bedeutung haben,
und Metall(thio)cyanaten der allgemeinen Formel (VII)
M-Q¹-CN (VII)
in welcher
Q¹ die die in einem der Ansprüche 1 bis 3 angegebene Bedeutung hat,
gegebenenfalls in Gegenwart eines Reaktionshilfsmittels und gegebenenfalls in Gegenwart eines Verdünnungsmittels umgesetzt werden,
oder daß
(d) substituierte Thiophen-3-sulfonsäurechloride der allgemeinen Formel (VI) 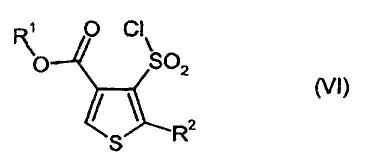 in welcher
R' und R² die in einem der Ansprüche 1 bis 3 angegebene Bedeutung haben,
mit Triazolin(thi)on-(thio)carboxamiden der allgemeinen Formel (VIII) 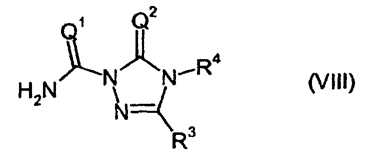 in welcher
Q¹, Q², R³ und R⁴ die in einem der Ansprüche 1 bis 3 angegebene Bedeutung haben,
gegebenenfalls in Gegenwart eines Reaktionshilfsmittels und gegebenenfalls in Gegenwart eines Verdünnungsmittels umgesetzt werden,
oder daß
(e) substituierte Thien-3-yl-sulfonylamino(thio)carbonylverbindungen der allgemeinen Formel (IX) 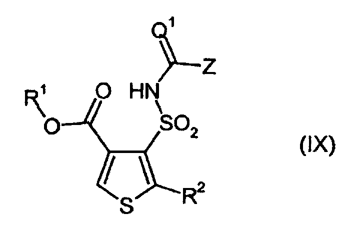 in welcher
Q¹, R¹ und R² die in einem der Ansprüche 1 bis 3 angegebene Bedeutung haben und
Z für Halogen, Alkoxy, Aryloxy oder Arylalkoxy steht,
mit Triazolin(thi)onen der allgemeinen Formel (V) 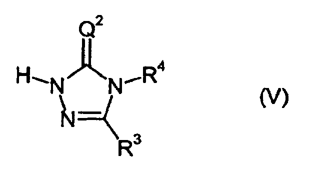 in welcher
Q², R⁴ und R⁵ die in einem der Ansprüche 1 bis 3 angegebene Bedeutung haben,
gegebenenfalls in Gegenwart eines Reaktionshilfsmittels und gegebenenfalls in Gegenwart eines Verdünnungsmittels umgesetzt werden,
und gegebenenfalls die nach den Verfahren (a), (b), (c), (d) oder (e) erhaltenen Verbindungen der Formel (I) nach üblichem Methoden in Salze überführt werden.

5. Verbindungen der allgemeinen Formel (II) 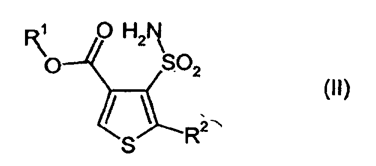 in welcher R¹ und R² die in einem der Ansprüche 1 bis 3 angegebene Bedeutung haben, ausgenommen die Verbindung 4-Methoxycarbonyl-thiophen-3-sulfonamid.

6. Verbindungen der allgemeinen Formel (VI) 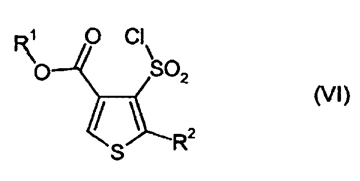 in welcher R¹ die in einem der Ansprüche 1 bis 3 angebene Bedeutung hat,
und
R² für Wasserstoff, Cyano, Nitro, für jeweils gegebenenfalls durch Cyano, Halogen oder C₁-C₄-Alkoxy substituiertes Alkyl, Alkoxy, Alkylthio, Alkylsulfinyl oder Alkylsulfonyl mit jeweils 1 bis 6 Kohlenstoffatomen in der Alkylgruppe, oder für jeweils gegebenenfalls durch Cyano oder Halogen substituiertes Alkenyl, Alkinyl, Alkenyloxy oder Alkinyloxy mit jeweils 2 bis 6 Kohlenstoffatomen in der Alkenyl- oder Alkinylgruppe steht,
ausgenommen die Verbindung 4-Methoxycarbonyl-thiophen-3-sulfonsäurechlorid.

7. Verfahren zum Bekämpfen von unerwünschtem Pflanzenwuchs, **dadurch gekennzeichnet, daß** man mindestens eine Verbindung gemäß einem der Ansprüche 1 bis 3 auf unerwünschte Pflanzen und/oder ihren Lebensraum einwirken läßt.

8. Verwendung von mindestens einer Verbindung gemäß einem der Ansprüche 1 bis 3 zum Bekämpfen von unerwünschten Pflanzen.

9. Herbizides Mittel, **gekennzeichnet durch** einen Gehalt an einer Verbindung gemäß einem der Ansprüche 1 bis 3 und üblichen Streckmitteln und/oder oberflächenaktiven Mitteln.

10. Verbindungen der Formel (I) gemäß Anspruch 1, bei denen eine in der folgenden Tabelle angegebenen Kombination der Reste Q¹, Q², R¹, R², R³ und R⁴ vorliegt:

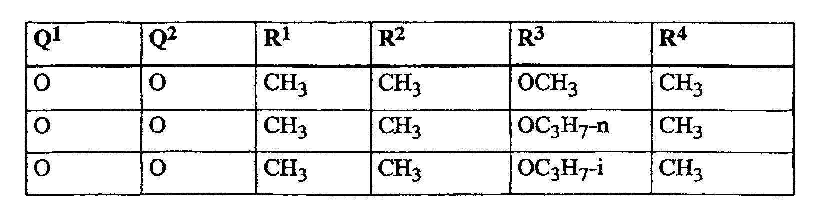

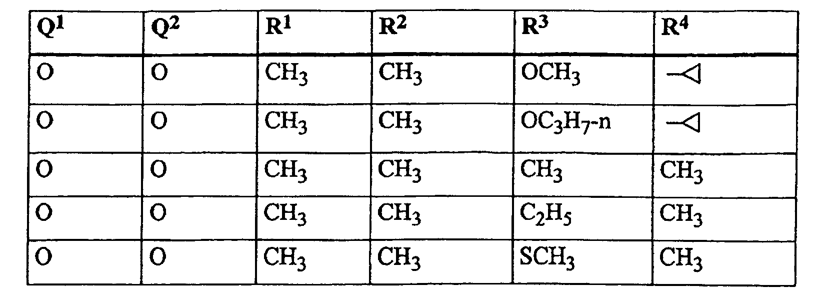

11. Die Verbindungen 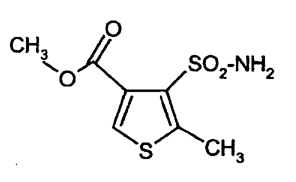 und

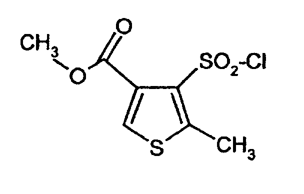

## Claims

1. Compounds of the general formula (I) 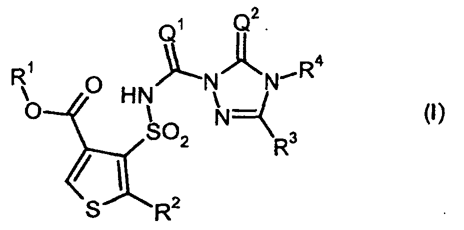 in which
Q¹ represents O (oxygen) or S (sulphur),
Q² represents O (oxygen) or S (sulphur),
R¹ represents optionally cyano-, halogen- or C₁-C₄-alkoxy-substituted alkyl having 1 to 6 carbon atoms, represents in each case optionally cyano- or halogen-substituted alkenyl or alkinyl having in each case 2 to 6 carbon atoms, represents in each case optionally cyano-, halogen- or C₁-C₄-alkyl-substituted cycloalkyl or cycloalkylalkyl having in each case 3 to 6 carbon atoms in the cycloalkyl group and optionally 1 to 4 carbon atoms in the alkyl moiety, represents in each case optionally nitro-, cyano-, halogen-, C₁-C₄-alkyl- or C₁-C₄-alkoxy-substituted aryl or arylalkyl having in each case 6 or 10 carbon atoms in the aryl group and optionally 1 to 4 carbon atoms in the alkyl moiety, or represents in each case optionally nitro-, cyano-, halogen-, C₁-C₄-alkyl- or C₁-C₄-alkoxy-substituted heterocyclyl or heterocyclylalkyl having in each case up to 6 carbon atoms and additionally 1 to 4 nitrogen atoms and/or 1 to 2 oxygen or sulphur atoms in the heterocyclyl group and optionally 1 to 4 carbon atoms in the alkyl moiety,
R² represents hydrogen, cyano, nitro, halogen, represents in each case optionally cyano-, halogen- or C₁-C₄-alkoxy-substituted alkyl, alkoxy, alkylthio, alkylsulphinyl or alkylsulphonyl having in each case 1 to 6 carbon atoms in the alkyl group, or represents in each case optionally cyano- or halogen-substituted alkenyl, alkinyl, alkenyloxy or alkinyloxy having in each case 2 to 6 carbon atoms in the alkenyl or alkinyl group,
R³ represents hydrogen, hydroxyl, mercapto, amino, cyano, fluorine, chlorine, bromine, iodine, represents optionally fluorine-, chlorine-, bromine-, cyano-, C₁-C₄-alkoxy-, C₁-C₄-alkyl-carbonyl- or C₁-C₄-alkoxy-carbonyl-substituted alkyl having 1 to 6 carbon atoms, represents in each case optionally fluorine-, chlorine- and/or bromine-substituted alkenyl or alkinyl having in each case 2 to 6 carbon atoms, represents in each case optionally fluorine-, chlorine-, cyano-, C₁-C₄-alkoxy- or C₁-C₄-alkoxy-carbonyl-substituted alkoxy, alkylthio, alkylamino or alkylcarbonylamino having in each case 1 to 6 carbon atoms in the alkyl group, represents alkenyloxy, alkinyloxy, alkenylthio, alkinylthio, alkenylamino or alkinylamino having in each case 3 to 6 carbon atoms in the alkenyl or alkinyl group, represents dialkylamino having in each case 1 to 4 carbon atoms in the alkyl groups, represents in each case optionally methyl- and/or ethyl-substituted aziridino, pyrrolidino, piperidino or morpholino, represents in each case optionally fluorine-, chlorine-, bromine-, cyano- and/or C₁-C₄-alkyl-substituted cycloalkyl, cycloalkenyl, cycloalkyloxy, cycloalkylthio, cycloalkylamino, cycloalkylalkyl, cycloalkylalkoxy, cycloalkylalkylthio or cycloalkylalkylamino having in each case 3 to 6 carbon atoms in the cycloalkyl or cycloalkenyl group and optionally 1 to 4 carbon atoms in the alkyl moiety, or represents in each case optionally fluorine-, chlorine-, bromine-, cyano-, nitro-, C₁-C₄-alkyl-, trifluoromethyl-, C₁-C₄-alkoxy- and/or C₁-C₄-alkoxy-carbonyl-substituted aryl, arylalkyl, aryloxy, arylalkoxy, arylthio, arylalkylthio, arylamino or arylalkylamino having in each case 6 or 10 carbon atoms in the aryl group and optionally 1 to 4 carbon atoms in the alkyl moiety, and
R⁴ represents hydrogen, hydroxyl, amino, cyano, represents C₂-C₁₀-alkylideneamino, represents optionally fluorine-, chlorine-, bromine-, cyano-, C₁-C₄-alkoxy-, C₁-C₄-alkyl-carbonyl- or C₁-C₄-alkoxy-carbonyl-substituted alkyl having 1 to 6 carbon atoms, represents in each case optionally fluorine-, chlorine- and/or bromine-substituted alkenyl or alkinyl having in each case 2 to 6 carbon atoms, represents in each case optionally fluorine-, chlorine-, bromine-, cyano-, C₁-C₄-alkoxy- or C₁-C₄-alkoxy-carbonyl-substituted alkoxy, alkylamino or alkylcarbonylamino having in each case 1 to 6 carbon atoms in the alkyl group, represents alkenyloxy having 3 to 6 carbon atoms, represents dialkylamino having in each case 1 to 4 carbon atoms in the alkyl groups, represents in each case optionally fluorine-, chlorine-, bromine-, cyano- and/or C₁-C₄-alkyl-substituted cycloalkyl, cycloalkylamino or cycloalkylalkyl having in each case 3 to 6 carbon atoms in the alkyl group and optionally 1 to 4 carbon atoms in the alkyl moiety, or represents in each case optionally fluorine-, chlorine-, bromine-, cyano-, nitro-, C₁-C₄-alkyl-, trifluoromethyl- and/or C₁-C₄-alkoxy-substituted aryl or arylalkyl having in each case 6 or 10 carbon atoms in the aryl group and optionally 1 to 4 carbon atoms in the alkyl moiety, or
R³ and R⁴ together represent optionally branched alkanediyl having 3 to 6 carbon atoms,
and the salts of these compounds.

2. Compounds according to Claim 1, **characterized in that**
Q¹ represents O (oxygen) or S (sulphur),
Q² represents O (oxygen) or S (sulphur),
R¹ represents in each case optionally cyano-, fluorine-, chlorine-, methoxy- or ethoxy-substituted methyl, ethyl, n- or i-propyl, n-, i-, s- or t-butyl, represents in each case optionally cyano-, fluorine- or chlorine-substituted propenyl, butenyl, propinyl or butinyl, represents in each case optionally cyano-, fluorine-, chlorine-, methyl- or ethyl-substituted cyclopropyl, cyclobutyl, cyclopentyl, cyclohexyl, cyclopropylmethyl, cyclobutylmethyl, cyclopentylmethyl or cyclohexylmethyl, represents in each case optionally cyano-, fluorine-, chlorine-, bromine-, methyl-, ethyl-, n- or i-propyl-, trifluoromethyl-, methoxy-, ethoxy-, n- or i-propoxy-, difluoromethoxy- or trifluoromethoxy-substituted phenyl, phenylmethyl or phenylethyl, or represents in each case optionally .cyano-, fluorine-, chlorine-, bromine-, methyl-, ethyl-, n- or i-propyl-, methoxy-, ethoxy-, n- or i-propoxy-substituted heterocyclyl or heterocyclylmethyl, where the heterocyclyl group is in each case selected from the group consisting of oxetanyl, thietanyl, furyl, tetrahydrofuryl, thienyl, tetrahydrothienyl,
R² represents hydrogen, cyano, fluorine, chlorine, bromine, represents in each case optionally cyano-, fluorine-, chlorine-, methoxy- or ethoxy-substituted methyl, ethyl, n- or i-propyl, n-, i-, s- or t-butyl, methoxy, ethoxy, n- or i-propoxy, methylthio, ethylthio, n- or i-propylthio, methylsulphinyl, ethylsulphinyl, methylsulphonyl or ethylsulphonyl, or represents in each case optionally cyano-, fluorine- or chlorine-substituted propenyl, butenyl, propinyl, butinyl, propenyloxy, butenyloxy, propinyloxy or butinyloxy,
R³ represents hydrogen, hydroxyl, mercapto, amino, cyano, fluorine, chlorine, bromine, represents in each case optionally fluorine-, chlorine-, cyano-, methoxy-, ethoxy-, n- or i-propoxy, acetyl-, propionyl-, n- or i-butyroyl-, methoxycarbonyl-, ethoxycarbonyl-, n- or i-propoxycarbonyl-substituted methyl, ethyl, n- or i-propyl, n-, i-, s- or t-butyl, represents in each case optionally fluorine-, chlorine- and/or bromine-substituted ethenyl, propenyl, butenyl, ethinyl, propinyl or butinyl, represents in each case optionally fluorine-, chlorine-, cyano-, methoxy-, ethoxy-, n- or i-propoxy-, methoxycarbonyl-, ethoxycarbonyl-, n- or i-propoxycarbonyl-substituted methoxy, ethoxy, n- or i-propoxy, n-, i-, s- or t-butoxy, methylthio, ethylthio, n- or i-propylthio, n-, i-, s- or t-butylthio, methylamino, ethylamino, n- or i-propylamino, n-, i-, s- or t-butylamino, acetylamino or propionylamino, represents propenyloxy, butenyloxy, ethinyloxy, propinyloxy, butinyloxy, propenylthio, butenylthio, propinylthio, butinylthio, propenylamino, butenylamino, propinylamino or butinylamino, represents dimethylamino, diethylamino or dipropylamino, represents in each case optionally fluorine-, chlorine-, methyl- and/or ethyl-substituted cyclopropyl, cyclobutyl, cyclopentyl, cyclohexyl, cyclopentenyl, cyclohexenyl, cyclopropyloxy, cyclobutyloxy, cyclopentyloxy, cyclohexyloxy, cyclopropylthio, cyclobutylthio, cyclopentylthio, cyclohexylthio, cyclopropylamino, cyclobutylamino, cyclopentylamino, cyclohexylamino, cyclopropylmethyl, cyclobutylmethyl, cyclopentylmethyl, cyclohexylmethyl, cyclopropylmethoxy, cyclobutylmethoxy, cyclopentylmethoxy, cyclohexylmethoxy, cyclopropylmethylthio, cyclobutylmethylthio, cyclopentylmethylthio, cyclohexylmethylthio, cyclopropylmethylamino, cyclobutylmethylamino, cyclopentylmethylamino or cyclohexylmethylamino, or represents in each case optionally fluorine-, chlorine-, bromine-, methyl-, trifluoromethyl-, methoxy- or methoxy-carbonyl-substituted phenyl, benzyl, phenoxy, benzyloxy, phenylthio, benzylthio, phenylamino or benzylamino, and
R⁴ represents hydrogen, hydroxyl, amino, represents in each case optionally fluorine-, chlorine-, cyano-, methoxy- or ethoxy-substituted methyl, ethyl, n- or i-propyl, n-, i-, s- or t-butyl, represents in each case optionally fluorine-, chlorine- and/or bromine-substituted ethenyl, propenyl, butenyl, propinyl or butinyl, represents in each case optionally fluorine-, chlorine-, cyano-, methoxy- or ethoxy-substituted methoxy, ethoxy, n- or i-propoxy, n-, i-, s- or t-butoxy, methylamino, ethylamino, n- or i-propylamino, n-, i-, s- or t-butylamino, represents propenyloxy or butenyloxy, represents dimethylamino or diethylamino, represents in each case optionally fluorine-, chlorine-, methyl- and/or ethyl-substituted cyclopropyl, cyclobutyl, cyclopentyl, cyclohexyl, cyclopropylamino, cyclobutylamino, cyclopentylamino, cyclohexylamino, cyclopropylmethyl, cyclobutylmethyl, cyclopentylmethyl or cyclohexylmethyl, or represents in each case optionally fluorine-, chlorine-, methyl-, trifluoromethyl- and/or methoxy-substituted phenyl or benzyl, or
R³ and R⁴ together represent trimethylene (propane-1,3-diyl), tetramethylene (butane-1,4-diyl) or pentamethylene (pentane-1,5-diyl),
and the sodium, potassium, magnesium, calcium, ammonium, C₁-C₄-alkyl-ammonium, di-(C₁-C₄-alkyl)-ammonium, tri-(C₁-C₄-alkyl)-ammonium, tetra-(C₁-C₄-alkyl) -ammonium, tri-(C₁-C₄-alkyl)-sulphonium, C₅-or C₆-cycloalkyl-ammonium and di-(C₁-C₂-alkyl)-benzylammonium salts of these compounds.

3. Compounds according to Claim 1, **characterized in that**
Q¹ represents O (oxygen),
Q² represents O (oxygen),
R¹ represents in each case optionally fluorine-, chlorine-, methoxy- or ethoxy-substituted methyl, ethyl, n- or i-propyl,
R² represents fluorine, chlorine, bromine or represents in each case optionally fluorine-, chlorine-, methoxy- or ethoxy-substituted methyl, ethyl, n- or i-propyl,
R³ represents hydrogen, chlorine, bromine, represents in each case optionally fluorine-, chlorine-, methoxy-, ethoxy-, n- or i-propoxy-substituted methyl, ethyl, n- or i-propyl, represents in each case optionally fluorine- or chlorine-substituted ethenyl, propenyl, butenyl, propinyl or butinyl, represents in each case optionally fluorine-, chlorine-, methoxy-, ethoxy-, n- or i-propoxy-substituted methoxy, ethoxy, n- or i-propoxy, methylthio, ethylthio, n- or i-propylthio, methylamino, ethylamino, n- or i-propylamino, represents propenyloxy, propinyloxy, propenylthio, propinylthio, propenylamino or propinylamino, represents dimethylamino or diethylamino, represents in each case optionally fluorine-, chlorine- or methyl-substituted cyclopropyl, cyclopropyloxy, cyclopropylmethyl or cyclopropylmethoxy, and
R⁴ represents in each case optionally fluorine-, chlorine-, methoxy- or ethoxy-substituted methyl, ethyl, n- or i-propyl, represents in each case optionally fluorine- or chlorine-substituted ethenyl, propenyl or propinyl, represents in each case optionally fluorine-, chlorine-, methoxy- or ethoxy-substituted methoxy, ethoxy, n- or i-propoxy, represents methylamino, or represents cyclopropyl,
and the sodium, potassium, magnesium, calcium, ammonium, C₁-C₄-alkyl-ammonium, di-(C₁-C₄-alkyl)-ammonium, tri- (C₁-C₄-alkyl) -ammonium, tetra- (C₁-C₄-alkyl)-ammonium, tri-(C₁-C₄-alkyl)-sulphonium, C₅-or C₆-cycloalkyl-ammonium and di- (C₁-C₂-alkyl)-benzylammonium salts of these compounds.

4. Process for preparing compounds according to any of Claims 1 to 3, **characterized in that**
(a) substituted thiophene-3-sulphonamides of the general formula (II) 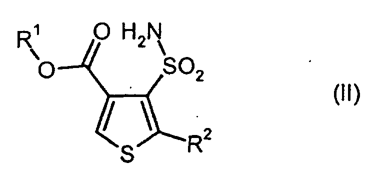 in which
R¹ and R² are each as defined in any of Claims 1 to 3
are reacted with substituted triazolin(ethi)ones of the general formula (III) 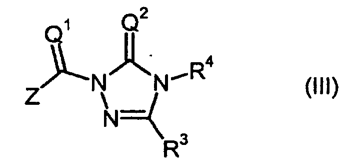 in which
Q¹, Q², R³ and R⁴ are each as defined in any of Claims 1 to 3 and
Z represents halogen, alkoxy, aryloxy or arylalkoxy,
if appropriate in the presence of a reaction auxiliary and if appropriate in the presence of a diluent,
or that
(b) substituted thien-3-yl-sulphonyl iso(thio)-cyanates of the general formula (IV) 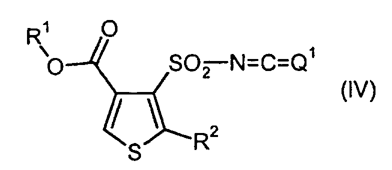 in which
Q¹, R¹ and R² are each as defined in any of Claims 1 to 3,
are reacted with triazolin(ethi)ones of the general formula (V) 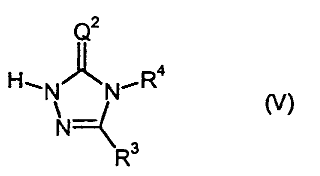 in which
Q², R⁴ and R⁵ are each as defined in any of Claims 1 to 3,
if appropriate in the presence of a reaction auxiliary and if appropriate in the presence of a diluent,
or that
(c) substituted thiophene-3-sulphonyl chlorides of the general formula (VI) 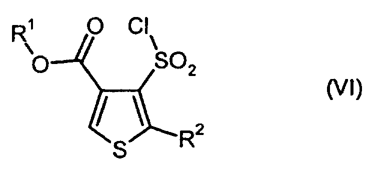 in which
R¹ and R² are each as defined in any of Claims 1 to 3,
are reacted with triazolin(ethi)ones of the general formula (V) 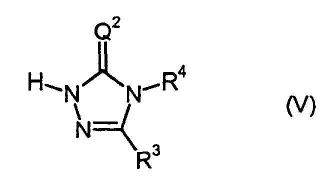 in which
Q², R⁴ and R⁵ are each as defined in any of Claims 1 to 3
and metal (thio)cyanates of the general formula (VII)
M-Q¹-CN (VII)
in which
Q¹ is as defined in any of Claims 1 to 3,
if appropriate in the presence of a reaction auxiliary and if appropriate in the presence of a diluent,
or that
(d) substituted thiophene-3-sulphonyl chlorides of the general formula (VI) 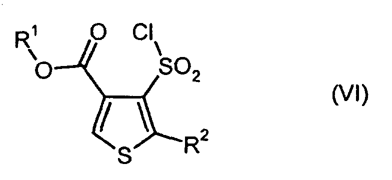 in which
R¹ and R² are each as defined in any of Claims 1 to 3
are reacted with triazolin(ethi)one-(thio)-carboxamides of the general formula (VIII) 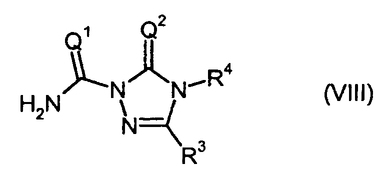 in which
Q¹, Q², R³ and R⁴ are each as defined in any of Claims 1 to 3,
if appropriate in the presence of a reaction auxiliary and if appropriate in the presence of a diluent,
or that
(e) substituted thien-3-yl-sulphonylamino(thio)-carbonyl compounds of the general formula (IX) 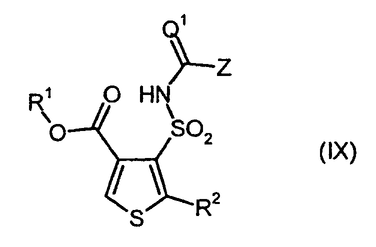 in which
Q¹, R¹ and R² are each as defined in any of Claims 1 to 3 and
Z represents halogen, alkoxy, aryloxy or arylalkoxy,
are reacted with triazolin(ethi)ones of the general formula (V) 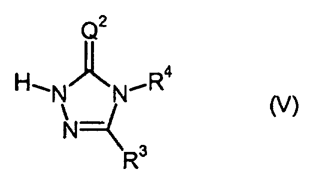 in which
Q², R⁴ and R⁵ are each as defined in any of Claims 1 to 3,
if appropriate in the presence of a reaction auxiliary and if appropriate in the presence of a diluent,
and the compounds of the formula (I) obtained by the processes (a), (b), (c); (d) or (e) are, if appropriate, converted by customary methods into salts.

5. Compounds of the general formula (II) 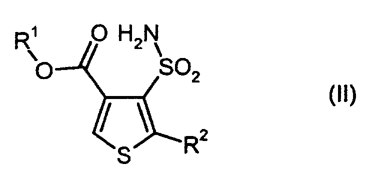 in which R¹ and R² are each as defined in any of Claims 1 to 3, except for the compound 4-methoxycarbonyl-thiophene-3-sulphonamide.

6. Compounds of the general formula (VI) 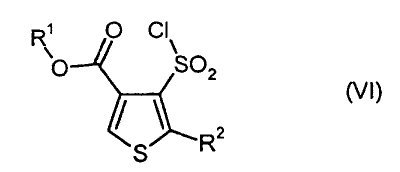 in which R¹ is as defined in any of Claims 1 to 3, and
R² represents hydrogen, cyano, nitro, halogen, represents in each case optionally cyano-, halogen- or C₁-C₄-alkoxy-substituted alkyl, alkoxy, alkylthio, alkylsulphinyl or alkylsulphonyl having in each case 1 to 6 carbon atoms in the alkyl group, or represents in each case optionally cyano- or halogen-substituted alkenyl, alkinyl, alkenyloxy or alkinyloxy having in each case 2 to 6 carbon atoms in the alkenyl or alkinyl group,
except for the compound 4-methoxycarbonylthiophene-3-sulphonyl chloride.

7. Method for controlling undesirable vegetation, **characterized in that** at least one compound according to any of Claims 1 to 3 is allowed to act on undesirable plants and/or their habitat.

8. Use of at least one compound according to any of Claims 1 to 3 for controlling undesirable plants.

9. Herbicidal compositions, **characterized in that** they comprise a compound according to any of Claims 1 to 3 and customary extenders and/or surfactants.

10. Compounds of the formula (I) according to Claim 1, in which one combination of the radicals Q¹, Q², R¹, R², R³ and R⁴ given in the following table is present:

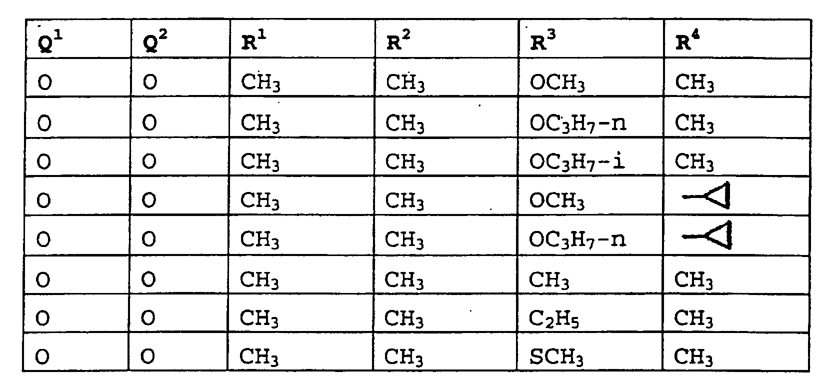

11. The compounds 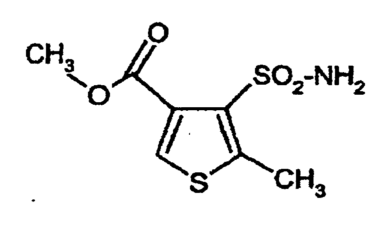 and

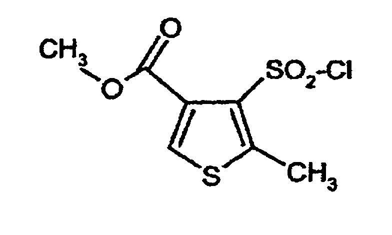

## Revendications

1. Composés de la formule générale (I) : 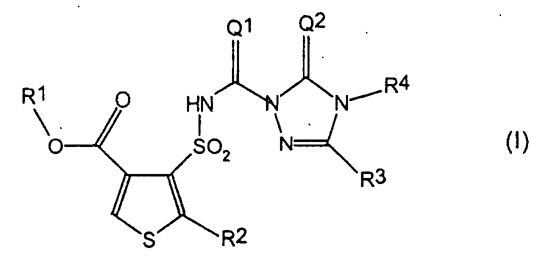 dans laquelle :
Q¹ représente O (oxygène) ou S (soufre) ;
Q² représente O (oxygène) ou S (soufre) ;
R¹ représente un radical alcoyle ayant 1 à 6 atomes de carbone, le cas échéant substitué par le radical cyano, un atome d'halogène ou un radical alcoxy en C₁-C₄, représente un radical alcényle ou alcynyle ayant chacun 2 à 6 atomes de carbone, le cas échéant substitué par le radical cyano ou un atome d'halogène, représente un radical cycloalcoyle ou cycloalcoylalcoyle ayant chacun, 3 à 6 atomes de carbone dans le radical cycloalcoyle et le cas échéant, 1 à 4 atomes de carbone dans la partie alcoyle, le cas échéant substitué par le radical cyano, un atome d'halogène ou un radical alcoyle en C₁-C₄, représente un radical aryle ou arylalcoyle ayant chacun, 6 à 10 atomes de carbone dans le radical aryle et le cas échéant, 1 à 4 atomes de carbone dans la partie alcoyle, le cas échéant substitué par le radical nitro, cyano, un atome d'halogène, un radical alcoyle en C₁-C₄ ou alcoxy en C₁-C₄, ou représente un radical hétérocyclyle ou hétérocyclylalcoyle ayant chacun, jusqu'à 6 atomes de carbone et en outre, 1 à 4 atomes d'azote et/ou 1 à 2 atomes d'oxygène ou de soufre dans le radical hétérocyclyle et le cas échéant, 1 à 4 atomes de carbone dans la partie alcoyle, le cas échéant substitué par le radical nitro, cyano, un atome d'halogène, un radical alcoyle en C₁-C₄ ou alcoxy en C₁-C₄ ;
R² représente l'atome d'hydrogène, le radical nitro, cyano, un atome d'halogène, un radical alcoyle, alcoxy, alcoylthio, alcoylsulfinyle ou alcoylsulfonyle ayant chacun, 1 à 6 atomes de carbone dans le radical alcoyle, le cas échéant substitué par le radical cyano, un atome d'halogène ou un radical alcoxy en C₁-C₄, ou représente un radical alcényle, alcynyle, alcényloxy ou alcynyloxy ayant chacun, 2 à 6 atomes de carbone dans le radical alcényle ou alcynyle, le cas échéant substitué par le radical cyano ou un atome d'halogène ;
R³ représente l'atome d'hydrogène, le radical hydroxyle, mercapto, amino, cyano, l'atome de fluor, de chlore, de brome, d'iode, un radical alcoyle ayant 1 à 6 atomes de carbone, le cas échéant substitué par l'atome de fluor, de chlore, de brome, le radical cyano, un radical alcoxy en C₁-C₄, (alcoyl en C₁-C₄)carbonyle ou (alcoxy en C₁-C₄) carbonyle, représente un radical alcényle ou alcynyle ayant chacun, 2 à 6 atomes de carbone, le cas échéant substitué par l'atome de fluor, de chlore et/ou de brome, représente un radical alcoxy, alcoylthio, alcoylamino ou alcoylcarbonylamino ayant chacun, 1 à 6 atomes de carbone dans le radical alcoyle, le cas échéant substitué par l'atome de fluor, de chlore, le radical cyano, un radical alcoxy en C₁-C₄, ou (alcoxy en C₁-C₄) carbonyle, représente un radical alcényloxy, alcynyloxy, alcénylthio, alcynylthio, alcénylamino ou alcynylamino ayant chacun, 3 à 6 atomes de carbone dans le radical alcényle ou alcynyle, représente un radical dialcoylamino ayant 1 à 4 atomes de carbone dans les radicaux alcoyle, représente le radical aziridino, pyrrolidino, pipéridino ou morpholino, chaque fois le cas échéant substitué par le radical méthyle et/ou éthyle, représente un radical cycloalcoyle, cycloalcényle, cycloalcoyloxy, cycloalcoylthio, cycloalcoylamino, cycloalcoylalcoyle, cycloalcoylalcoxy, cycloalcoylalcoylthio ou cycloalcoylalcoylamino, ayant chacun, 3 à 6 atomes de carbone dans le radical cycloalcoyle ou cycloalcényle et le cas échéant, 1 à 4 atomes de carbone dans la partie alcoyle, le cas échéant substitué par l'atome de fluor, de chlore, de brome, le radical cyano et/ou un radical alcoyle en C₁-C₄, ou représente un radical aryle, arylalcoyle, aryloxy, arylalcoxy, arylthio, arylalcoylthio, arylamino ou arylalcoylamino, ayant chacun, 6 ou 10 atomes de carbone dans le radical aryle et le cas échéant, 1 à 4 atomes de carbone dans la partie alcoyle, le cas échéant substitué par l'atome de fluor, de chlore, de brome, le radical cyano, nitro, un radical alcoyle en C₁-C₄, le radical trifluorométhyle, un radical alcoxy en C₁-C₄ et/ou un radical (alcoxy en C₁-C₄) carbonyle, et
R⁴ représente l'atome d'hydrogène, le radical hydroxyle, amino, cyano, un radical alcoylidènamino en C₂-C₁₀, représente un radical alcoyle ayant 1 à 6 atomes de carbone, le cas échéant substitué par l'atome de fluor, de chlore, de brome, le radical cyano, un radical alcoxy en C₁-C₄, (alcoyl en C₁-C₄) carbonyle ou (alcoxy en C₁-C₄)carbonyle, représente un radical alcényle ou alcynyle ayant chacun, 2 à 6 atomes de carbone, le cas échéant substitué par l'atome de fluor, de chlore et/ou de brome, représente un radical alcoxy, alcoylamino ou alcoylcarbonylamino ayant chacun, 1 à 6 atomes de carbone dans le radical alcoyle, le cas échéant substitué par l'atome de fluor, de chlore, le radical cyano, un radical alcoxy en C₁-C₄, ou (alcoxy en C₁-C₄) carbonyle, représente un radical alcényloxy, ayant 3 à 6 atomes de carbone, représente un radical dialcoylamino ayant 1 à 4 atomes de carbone dans les radicaux alcoyle, représente un radical cycloalcoyle, cycloalcoylamino ou cycloalcoylalcoyle, ayant chacun, 3 à 6 atomes de carbone dans le radical cycloalcoyle et le cas échéant, 1 à 4 atomes de carbone dans la partie alcoyle, le cas échéant substitué par l'atome de fluor, de chlore, de brome, le radical cyano et/ou un radical alcoyle en C₁-C₄, ou représente un radical aryle ou arylalcoyle, ayant chacun, 6 ou 10 atomes de carbone dans le radical aryle et le cas échéant, 1 à 4 atomes de carbone dans la partie alcoyle, le cas échéant substitué par l'atome de fluor, de chlore, de brome, le radical cyano, nitro, un radical alcoyle en C₁-C₄, le radical trifluorométhyle et/ou un radical alcoxy en C₁-C₄ ;
R³ et R⁴ représentent ensemble un alcanediyle le cas échéant ramifié, ayant 3 à 6 atomes de carbone ;
ainsi que les sels de ces composés.

2. Composés suivant la revendication 1, **caractérisés en ce que** :
Q¹ représente O (oxygène) ou S (soufre) ;
Q² représente O (oxygène) ou S (soufre) ;
R¹ représente le radical méthyle, éthyle, n- ou i-propyle, n-, i-, s- ou t-butyle, le cas échéant substitué par le radical cyano, l'atome de fluor, de chlore, le radical méthoxy ou éthoxy, représente le radical propényle, butényle, propynyle ou butynyle, le cas échéant substitué par le radical cyano ou l'atome de fluor ou de chlore, représente le radical cyclopropyle, cyclobutyle, cyclopentyle, cyclohexyle, cyclopropylméthyle, cyclobutylméthyle, cyclopentylméthyle ou cyclohexylméthyle, le cas échéant substitué par le radical cyano, l'atome de fluor, de chlore, le radical méthyle ou éthyle, représente le radical phényle, phénylméthyle ou phényléthyle, le cas échéant substitué par le radical cyano, l'atome de fluor, de chlore, de brome, le radical méthyle, éthyle, n- ou i-propyle, trifluorométhyle, méthoxy, éthoxy, n- ou i-propoxy, difluorométhoxy ou trifluorométhoxy, ou représente un radical hétérocyclyle ou hétérocyclylalcoyle, le cas échéant substitué par le radical cyano, l'atome de fluor, de chlore, de brome, le radical méthyle, éthyle, n- ou i-propyle, méthoxy, éthoxy, n- ou i-propoxy, où le radical hétérocyclyle est chaque fois choisi parmi la série des radicaux oxétannyle, thiétannyle, furyle, tétrahydrofuryle, thiényle, tétrahydrothiényle ;
R² représente l'atome d'hydrogène, le radical cyano, l'atome de fluor, de chlore, de brome, représente le radical méthyle, éthyle, n- ou i-propyle, n-, i-, s- ou t-butyle, méthoxy, éthoxy, n- ou i-propoxy, méthylthio, éthylthio, n- ou i-propylthio, méthylsulfinyle, éthylsulfinyle, méthylsulfonyle ou éthylsulfonyle, le cas échéant substitué par le radical cyano, l'atome de fluor, de chlore, le radical méthoxy ou éthoxy, ou représente le radical propényle, butényle, propynyle, butynyle, propényloxy, butényloxy, propynyloxy ou butynyloxy, le cas échéant substitué par le radical cyano, l'atome de fluor ou de chlore ;
R³ représente l'atome d'hydrogène, le radical hydroxyle, mercapto, amino, cyano, l'atome de fluor, de chlore, de brome, représente le radical méthyle, éthyle, n- ou i-propyle, n-, i-, s- ou t-butyle, le cas échéant substitué par l'atome de fluor, de chlore, le radical cyano, le radical méthoxy, éthoxy, n- ou i-propoxy, acétyle, propionyle, n- ou -i-butyroyle, méthoxycarbonyle, éthoxycarbonyle, n- ou i-propoxycarbonyle, représente le radical éthényle, propényle, butényle, éthynyle, propynyle ou butynyle, le cas échéant substitué par l'atome de fluor, de chlore et/ou de brome, représente le radical méthoxy, éthoxy, n- ou i-propoxy, n-, i-, s- ou t-butoxy, méthylthio, éthylthio, n- ou i-propylthio, n-, i-, sou t-butylthio, méthylamino, éthylamino, n- ou i-propylamino, n-, i-, s- ou t-butylamino, acétylamino ou propionylamino, le cas échéant substitué par l'atome de fluor, de chlore, le radical cyano, le radical méthoxy, éthoxy, n- ou i-propoxy, méthoxycarbonyle, éthoxycarbonyle, n- ou i-propoxycarbonyle, représente le radical propényloxy, butényloxy, éthynyloxy, propynyloxy, butynyloxy, propénylthio, buténylthio, propynylthio ou butynylthio, propénylamino, buténylamino, propynylamino ou butynylamino, représente le radical diméthylamino, diéthylamino ou dipropylamino représente un radical cyclopropyle, cyclobutyle, cyclopentyle, cyclohexyle, cyclopentényle, cyclohexényle, cyclopropyloxy, cyclobutyloxy, cyclopentyloxy, cyclohexyloxy, cyclopropylthio, cyclobutylthio, cyclopentylthio, cyclohexylthio, cyclopropylamino, cyclobutylamino, cyclopentylamino, cyclohexylamino, cyclopropylméthyle, cyclobutylméthyle, cyclopentylméthyle, cyclohexylméthyle, cyclopropylméthoxy, cyclobutylméthoxy, cyclopentylméthoxy, cyclohexylméthoxy, cyclopropylméthylthio, cyclobutylméthylthio, cyclopentylméthylthio, cyclohexylméthylthio, cyclopropylméthylamino, cyclobutylméthylamino, cyclopentylméthylamino ou cyclohexylméthylamino, le cas échéant substitué par l'atome de fluor, de chlore, le radical méthyle et/ou éthyle, ou représente le radical phényle, benzyle, phénoxy, benzyloxy, phénylthio, benzylthio, phénylamino ou benzylamino, le cas échéant substitué par l'atome de fluor, de chlore, de brome, le radical méthyle, trifluorométhyle, méthoxy ou méthoxycarbonyle, et
R⁴ représente l'atome d'hydrogène, le radical hydroxyle, amino, le radical méthyle, éthyle, n- ou i-propyle, n-, i-, s- ou t-butyle, le cas échéant substitué par l'atome de fluor, de chlore, le radical cyano, le radical méthoxy ou éthoxy, représente le radical éthényle, propényle, butényle, propynyle ou butynyle, le cas échéant substitué par l'atome de fluor, de chlore et/ou de brome, représente le radical méthoxy, éthoxy, n- ou i-propoxy, n-, i-, s- ou t-butoxy, méthylamino, éthylamino, n- ou i-propylamino, n-, i-, s- ou t-butylamino, le cas échéant substitué par l'atome de fluor, de chlore, le radical cyano, le radical méthoxy ou éthoxy, représente le radical propényloxy ou butényloxy, représente le radical diméthylamino ou diéthylamino, représente le radical cyclopropyle, cyclobutyle, cyclopentyle, cyclohexyle, cyclopropylamino, cyclobutylamino, cyclopentylamino, cyclohexylamino, cyclopropylméthyle, cyclobutylméthyle, cyclopentylméthyle ou cyclohexylméthyle, le cas échéant substitué par l'atome de fluor, de chlore, le radical méthyle et/ou éthyle, ou représente le radical phényle ou benzyle, le cas échéant substitué par l'atome de fluor, de chlore, de brome, le radical méthyle, trifluorométhyle et/ou méthoxy, ou
R³ et R⁴ représentent ensemble le radical triméthylène (propane-1,3-diyle), tétraméthylène (butane-1,4-diyle) ou pentaméthylène (pentane-1,5-diyle) ;
ainsi que les sels de sodium, de potassium, de magnésium, de calcium, d'ammonium, d'(alcoyl en C₁-C₄) ammonium, de di(alcoyl en C₁-C₄) ammonium, de tri(alcoyl en C₁-C₄) ammonium, de tétra(alcoyl en C₁-C₄) ammonium, de tri(alcoyl en C₁-C₄)sulfonium, de (cycloalcoyl en C₅ ou C₆) ammonium et de di (alcoyl en C₁-C₂)benzylammonium de ces composés.

3. Composés suivant la revendication 1, **caractérisés en ce que** :
Q¹ représente O (oxygène) ;
Q² représente O (oxygène) ;
R¹ représente le radical méthyle, éthyle, n- ou i-propyle, le cas échéant substitué par l'atome de fluor, de chlore, le radical méthoxy ou éthoxy ;
R² représente l'atome de fluor, de chlore, de brome, ou le radical méthyle, éthyle, n- ou i-propyle, le cas échéant substitué par l'atome de fluor, de chlore, le radical méthoxy ou éthoxy ;
R³ représente l'atome d'hydrogène, de chlore, de brome, le radical méthyle, éthyle, n- ou i-propyle, le cas échéant substitué par l'atome de fluor, de chlore, le radical méthoxy, éthoxy, n- ou i-propoxy, représente le radical éthényle, propényle, butényle, propynyle ou butynyle, le cas échéant substitué par l'atome de fluor ou de chlore, représente le radical méthoxy, éthoxy, n- ou i-propoxy, méthylthio, éthylthio, n- ou i-propylthio, méthylamino, éthylamino, n- ou i-propylamino, le cas échéant substitué par l'atome de fluor, de chlore, le radical méthoxy, éthoxy, n- ou i-propoxy, représente le radical propényloxy, propynyloxy, propénylthio, propynylthio, propénylamino, ou propynylamino, représente le radical diméthylamino ou diéthylamino, représente le radical cyclopropyle, cyclopropyloxy, cyclopropylméthyle ou cyclopropylméthoxy, le cas échéant substitué par l'atome de fluor, de chlore ou le radical méthyle, et
R⁴ représente le radical méthyle, éthyle, n- ou i-propyle, le cas échéant substitué par l'atome de fluor, de chlore, le radical méthoxy ou éthoxy, représente le radical éthényle, propényle ou propynyle, le cas échéant substitué par l'atome de fluor ou de chlore, représente le radical méthoxy, éthoxy, n- ou i-propoxy, le cas échéant substitué par l'atome de fluor, de chlore, le radical méthoxy ou éthoxy, représente le radical méthylamino, ou représente le radical cyclopropyle ;
ainsi que les sels de sodium, de potassium, de magnésium, de calcium, d'ammonium, d'(alcoyl en C₁-C₄) ammonium, de di (alcoyl en C₁-C₄) ammonium, de tri (alcoyl en C₁-C₄)ammonium, de tétra(alcoyl en C₁-C₄) ammonium, de tri (alcoyl en C₁-C₄) sulfonium, de (cycloalcoyl en C₅ ou C₆) ammonium et de di (alcoyl en C₁-C₂)benzylammonium de ces composés.

4. Procédé de préparation de composés suivant l'une quelconque des revendications 1 à 3, **caractérisé en ce que** :
(a) on fait réagir des thiophène-3-sulfonamides substitués de la formule générale (II) : 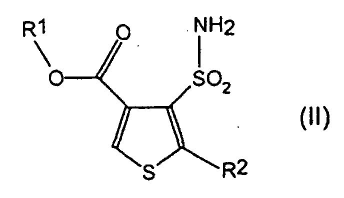 dans laquelle :
R¹ et R² ont la signification donnée dans l'une quelconque des revendications 1 à 3,
avec des triazoline(thi)ones substituées de la formule générale (III) : 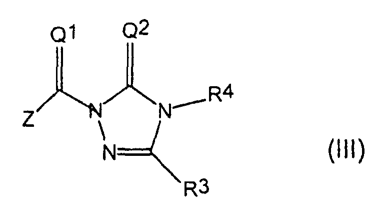 dans laquelle :
Q¹, Q², R³ et R⁴ ont la signification donnée dans l'une quelconque des revendications 1 à 3, et
Z représente un atome d'halogène, un radical alcoxy, aryloxy ou arylalcoxy,
le cas échéant en présence d'un auxiliaire de réaction et le cas échéant, en présence d'un agent de dilution,
ou **en ce que**
(b) on fait réagir des thièn-3-ylsulfonyl-iso(thio)cyanates substitués de la formule générale (IV) : 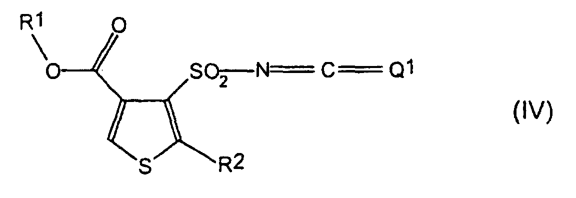 dans laquelle :
Q¹, R¹ et R² ont la signification donnée dans l'une quelconque des revendications 1 à 3,
avec des triazoline(thi)ones substituées de la formule générale (V) : 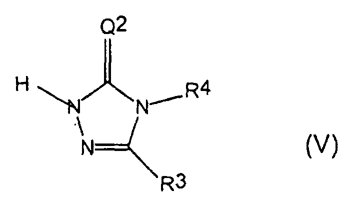 dans laquelle :
Q², R³ et R⁴ ont la signification donnée dans l'une quelconque des revendications 1 à 3, et
le cas échéant en présence d'un auxiliaire de réaction et le cas échéant, en présence d'un agent de dilution,
ou **en ce que**
(c) on fait réagir des chlorures d'acide thiophène-3-sulfonique substitués de la formule générale (VI) : 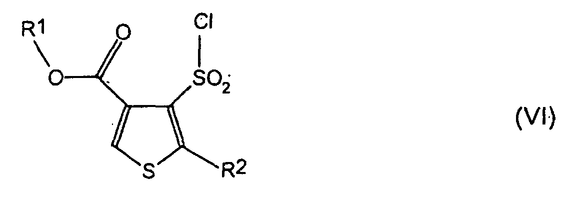 dans laquelle :
R¹ et R² ont la signification donnée dans l'une quelconque des revendications 1 à 3,
avec des triazoline(thi)ones substituées de la formule générale (V) : 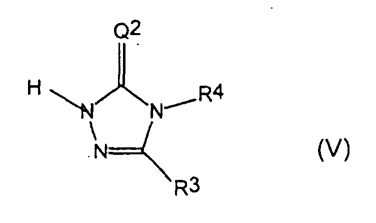 dans laquelle :
Q², R³ et R⁴ ont la signification donnée dans l'une quelconque des revendications 1 à 3, et
des (thio)cyanates métalliques de la formule générale (VII) :
M - Q¹ - CN (VII)
dans laquelle :
Q¹ a la signification donnée dans l'une quelconque des revendications 1 à 3,
le cas échéant en présence d'un auxiliaire de réaction et le cas échéant, en présence d'un agent de dilution,
ou **en ce que**
(d) on fait réagir des chlorures d'acide thiophène-3-sulfonique substitués de la formule générale (VI) : 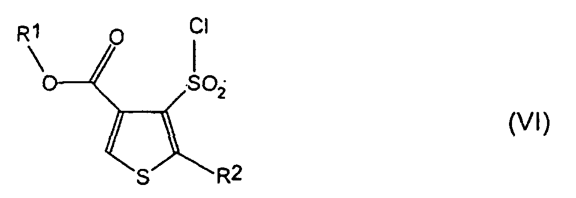 dans laquelle :
R¹ et R² ont la signification donnée dans l'une quelconque des revendications 1 à 3,
avec des triazoline(thi)one-(thio)carboxamides substituées de la formule générale (VIII) : 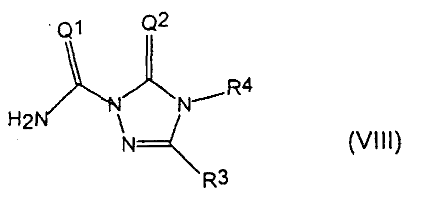 dans laquelle :
Q¹, Q², R³ et R⁴ ont la signification donnée dans l'une quelconque des revendications 1 à 3,
le cas échéant en présence d'un auxiliaire de réaction et le cas échéant, en présence d'un agent de dilution,
ou **en ce que**
(e) on fait réagir des composés thièn-3-ylsulfonylamino(thio)carbonylés substitués de la formule générale (IX) : 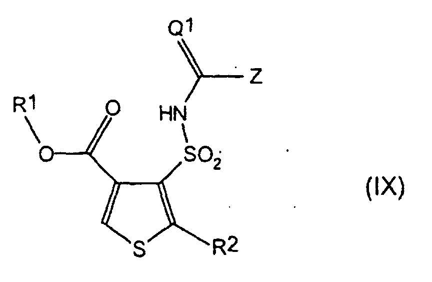 dans laquelle :
Q¹, R¹ et R² ont la signification donnée dans l'une quelconque des revendications 1 à 3,
Z représente un atome d'halogène, un radical alcoxy, aryloxy ou arylalcoxy,
avec des triazoline(thi)ones substituées de la formule générale (V) : 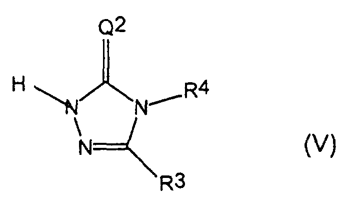 dans laquelle :
Q², R³ et R⁴ ont la signification donnée dans l'une quelconque des revendications 1 à 3, et
le cas échéant en présence d'un auxiliaire de réaction et le cas échéant, en présence d'un agent de dilution,
et le cas échéant, les composés de la formule (I), obtenus selon les procédés (a), (b), (c), (d) ou (e) sont convertis en sels par les procédés usuels.

5. Composés de la formule générale (II) : 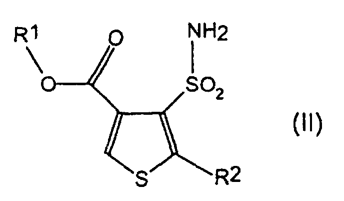 dans laquelle :
R¹ et R² ont la signification donnée dans l'une quelconque des revendications 1 à 3, à l'exception du composé 4-méthoxycarbonylthiophène-3-sulfonamide.

6. Composés de la formule générale (VI) : 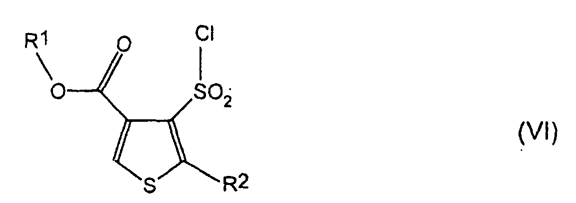 dans laquelle :
R¹ a la signification donnée dans l'une quelconque des revendications 1 à 3, et
R² représente l'atome d'hydrogène, le radical cyano, nitro, un radical alcoyle, alcoxy, alcoylthio, alcoylsulfinyle ou alcoylsulfonyle ayant chacun, 1 à 6 atomes de carbone dans le radical alcoyle, le cas échéant substitué par le radical cyano, un atome d'halogène ou un radical alcoxy en C₁-C₄, ou représente un radical alcényle, alcynyle, alcényloxy ou alcynyloxy ayant chacun, 2 à 6 atomes de carbone dans le radical alcényle ou alcynyle, le cas échéant substitué par le radical cyano ou un atome d'halogène ;
à l'exception du composé chlorure d'acide 4-méthoxycarbonylthiophène-3-sulfonique.

7. Procédé pour lutter contre la croissance non désirée de végétaux, **caractérisé en ce que** l'on fait agir au moins un composé suivant l'une quelconque des revendications 1 à 3 sur les végétaux non souhaités et/ou leur biotope.

8. Utilisation d'au moins un composé suivant l'une quelconque des revendications 1 à 3, pour lutter contre les végétaux non désirés

9. Agent herbicide, **caractérisé par** une teneur en au moins un composé suivant l'une quelconque des revendications 1 à 3, avec les diluants et/ou agents tensioactifs usuels.

10. Composés de la formule (I) suivant la revendication 1, dans lesquels une des combinaisons du tableau suivant, des restes Q¹, Q², R¹, R², R³ et R⁴ est présente :

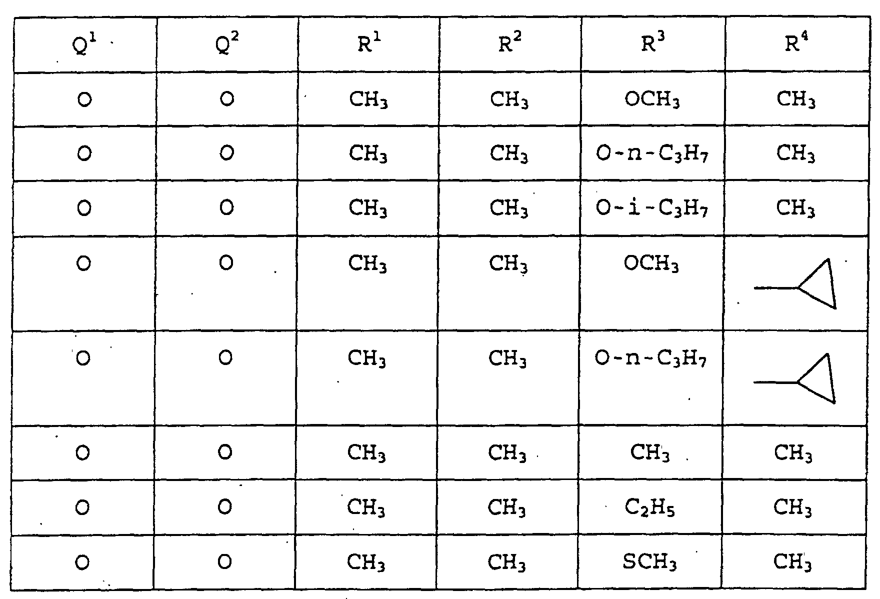

11. Les composés: 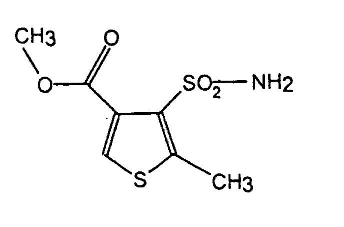 et
